# EUROPEAN PATENT APPLICATION

(11) **EP 4 075 137 A1**
(43) Date of publication of application: **19.10.2022**
(21) Application number: 20896495.7
(22) Date of filing: 23.11.2020
(51) Int. Cl.: G01N 33/574, G01N 33/68, C12Q 1/6886, C12N 5/09, G16H 70/60, G01N 33/569

(54) **PREPARATION DEVICE AND PREPARATION METHOD FOR EXOSOME LIQUID BIOPSY SAMPLE AND METHOD FOR ANALYZING EXOSOME LIQUID BIOPSY SAMPLE PREPARED THEREBY**

(30) Priority: 02.12.2019 KR 20190158298; 29.06.2020 KR 20200079415
(71) Applicant: Sol Bio Corporation, Seoul 04780 (KR)
(72) Inventor: PAEK, Sung-Ho, Seoul 04968 (KR); CHOI, Da-Yeon, Namyangju-Si Gyeonggi-do 12014 (KR)
(74) Representative: Hoefer & Partner Patentanwälte mbB
(86) International application number: PCT/KR2020/016553
(87) International publication number: WO 2021/112464

(57) **Abstract**

The present invention relates to a technique in which a target exosome subpopulation, a sub-subpopulation, or a lower population in human fluid, which is associated with a specific disease, is isolated and recovered in its intact form at high yield to prepare a liquid biopsy sample and analyzed.

## Description

### Technical Field

The present invention relates to a technology in which a liquid biopsy sample is prepared by separating a population sample into a subpopulation, a sub-subpopulation or a subset thereof containing exosomes associated with a specific disease such as a cancer disease and is analyzed.

### Background Art

Recently, the concept of "liquid biopsy" has been introduced to minimize pain during screening for specific diseases such as cancer and degenerative diseases and to achieve early diagnosis of the diseases. Liquid biopsy refers to a non-invasive method for extracting body fluids, including blood, saliva, and urine, in a relatively simple manner and analyzing specific disease-associated cells or components (e.g., peptides, proteins, nucleic acids, organelles, and specific substances) released therefrom in the body fluids. Liquid biopsy helps to ascertain the occurrence of specific diseases at an early stage. For this reason, liquid biopsy has attracted attention as an alternative to existing test methods such as diagnostic imaging, tissue examination, and blood testing.

Generally, liquid biopsy for diagnosing specific diseases, for example, cancer diseases, is advantageous over tissue biopsy. First, liquid biopsy using body fluids is relatively easy to perform and reduces the risk of complications. In contrast, tissue biopsy usually involves expensive, invasive surgical tissue sampling that poses potential risks. Second, liquid biopsy enables real-time continuous monitoring of the progression of cancer diseases. In contrast, tissue biopsy only provides a snapshot of a single disease lesion when necessary and fails to monitor the progression and treatment of diseases every moment. Third, liquid biopsy collects biomaterials generated from tumor cells, allowing a more comprehensive analysis of the composition of the corresponding tumor at the molecular level. In contrast, tissue biopsy collects diverse tumors, making it difficult to fully understand the composition of a target tumor. Fourth, liquid biopsy enables multiple sampling from various body fluids as needed. In contrast, the limited size of samples for tissue biopsy makes it impossible to divide and use them for several diagnostic purposes. Fifth, liquid biopsy can provide reliable results for clinical decision making due to its relatively high sensitivity (measurement accuracy for positive samples) and specificity (measurement accuracy for negative samples). In contrast, the restricted accuracy of tissue biopsy limits the clinical usefulness of test results.

Circulating tumor cells (CTCs), cell-free DNA, and exosomes have been recognized for their potential as biomarkers for liquid biopsy in the field of cancer disease diagnosis. Although circulating tumor cells are excellent targets in terms of enrichment, selectivity, and stability of isolated nucleic acids, their extremely low blood level makes it difficult to use them for early diagnosis of cancer diseases. Cell-free DNA is difficult to enrich and select despite its high blood level and their stability after isolation is low. Moreover, due to the presence of a large amount of non-specific DNA that is not associated with target diseases, overcoming the problem of noise during analysis is becoming a major issue. Compared to these two types of biomarkers, exosomes are abundantly present in the blood and are easy to enrich and select. In addition, the state of nucleic acids isolated from exosomes is stable. However, since the size and density of exosome particles and the compositions of protein and nucleic acid components present in exosome particles are extremely heterogeneous, only disease-associated exosomes need to be isolated for liquid biopsy.

Exosomes have received attention as biomarkers for liquid biopsy. Exosomes are released in the form of microvesicles (MVEs) from human cells and reflect the characteristics of the original cells. Exosomes are 30-150 nm in size and can be extracted from various body fluids such as blood, saliva, and urine. Since exosomes are vesicles composed of lipid bilayers, they are not only stable *in vivo* but also free from the attack of protease, RNase, and DNase. Under these circumstances, there has been increasing research on the use of exosomes as new biomarkers for diagnosing specific diseases such as cancer and degenerative diseases. In practice, products using components (e.g., proteins and RNAs) present in exosomes as markers have been developed or are currently being developed for early diagnosis and companion diagnostics of cancer.

The following exosomal proteins and RNA biomarkers can be used to diagnose specific diseases, particularly cancer diseases, during liquid biopsy. First, considering exosome biosynthesis and processing, smaller MVEs such as exosomes bud into cells to form small intracellular vesicles containing intracytoplasmic proteins, mRNAs, and microRNAs (miRNAs). Second, such intracellular vesicles are released as exosomes to the outside when MVEs fuse with the cell membrane. Third, the released exosomes start to move towards destinations determined by the binding of specific ligands on their surface. Fourth, the exosomes reaching target cells enter the target cells via the endocytosis pathway or fuse with the target cell membrane to directly release their components into the cytoplasm.

Protein components involved in exosome biosynthesis in the first stage include hepatocyte growth factor-regulated tyrosine kinase substrate (HRS), signal transducing adapter molecule (STAM1), tumor susceptibility gene (TSG101), and CD81. Examples of known proteins involved in exosome release in the second stage include RAB2B, RAB5A, RAB7, and RAB9A. Theses protein molecules are not only potential target candidates for the development of therapeutic agents but also can be used as major targets in the development of techniques for detection and isolation of exosomes.

Exosomes released into body fluids contain various cellular constituent components such as double-stranded DNAs, RNAs, proteins, and lipids. According to a recent study, DNA molecules loaded in exosomes are representative of the entire genome and can reflect the mutational status of tumor cells from which exosomes are released. Accordingly, analysis of double-stranded DNA molecules in exosomes would be particularly useful for early detection of cancer diseases or metastases. The composition of proteins in exosomes varies depending on cells or tissues from which exosomes are released, but most exosomes contain general assembly of evolutionarily conserved protein molecules. The protein content of exosomes is important for the development of techniques and methods for the isolation, detection, and analysis of exosomes. Furthermore, protein molecules of exosomes can be used as biomarkers in information collection and diagnosis of other pathological conditions. Since different contents of cell- and exosome-derived mRNAs and miRNAs and functionalities of miRNAs in exosomes were reported, the results of several studies on miRNAs have been published simultaneously and have triggered explosive research on exosomes. In practice, miRNAs have attracted attention as special targets in the development of therapeutics agents as well as diagnostic agents. Many studies based on new detection techniques reported that miRNAs are found in almost all body fluids. Thus, miRNAs have emerged as potential biomarkers for diagnostic analysis. In practice, analysis of various body fluid samples revealed that a large number of different miRNAs are found depending on the sources of the samples.

Particularly, exosomes are known to play a role in tumorigenesis, cancer metastasis, and drug resistance as key cancer-related issues. In the microenvironment where tumors arise, tumor cells release exosomes and other MVEs that contribute to the migration of tumor cells during tumor progression and metastasis when angiogenesis is stimulated, like other cells. Tumor-derived vesicles also contain molecules involved in immunosuppression. These molecules can inactivate T lymphocytes or natural killer cells or promote the differentiation of regulatory T lymphocytes or myeloid cells to suppress immune responses. It was also reported that exosomes released from tumor cells cause metastasis of tumor activity.

However, since exosomes derived from all cells exist in a mixed state in body fluids, there is a limitation in accurately diagnosing specific diseases such as cancer from a bulk population of exosome samples separated from body fluids. Moreover, the enrichment of exosomes derived from specific disease-associated cells in body fluids is relatively very low in the early stage of disease onset, isolation and enrichment of target exosomes from body fluids are prerequisites for early diagnosis. Many techniques for exosome isolation are known. Of these, ultracentrifugation based on a density difference is adopted as the gold standard for exosome isolation due to its high reproducibility and relatively stable process. However, ultracentrifugation has disadvantages in that the use of an expensive system is required and it takes a long time for isolation. In attempts to overcome these disadvantages, methods for isolating exosomes by precipitation and methods for isolating exosomes by size exclusion chromatography or on the basis of size using microfluidics have been developed.

However, the existing exosome isolation techniques are limited in selectively detecting trace amounts of exosomes derived from specific cells such as cancer cells because all exosomes present in body fluids are provided in the form of single populations. In practice, various biochemical components (including membrane proteins, internalized proteins, and RNAs) present in the membrane or inside of exosomes released from cells are very heterogeneous in their composition. Particularly, it was reported that specific exosomes derived from cancer cells contain mRNAs or miRNAs involved in the creation of local environments in the initial stage of angiogenesis and cancer metastasis. The specific exosomes are also known to contain biochemical components involved in the induction of cytotoxicity of immune cells or the transformation of normal cells into cancer cells during cancer metastasis.

In the case where a bulk population containing all exosomes present in a body fluid is used as a liquid biopsy sample for the diagnosis of a specific disease such as cancer, high diagnostic accuracy is very difficult to achieve because sample matrix effects (e.g., non-specific binding of non-specific exosomes) cause negative results in analytical specificity and sensitivity. In an attempt to solve such analytical problems, multiple biomarkers (e.g., a plurality of different miRNA species) known to increase in a body fluid of a patient with a specific disease can be measured simultaneously to determine whether the disease occurs or not despite their low specificities. However, the use of the multiple biomarkers has limited synergistic effects because most exosomal markers are non-specifically associated with the disease and their specificity for the disease is fundamentally low.

Thus, there is an urgent need for a solution to the problem that exosomes associated with the occurrence of a specific disease such as cancer cannot be selectively isolated and used to diagnose the specific disease.

### Detailed Description of the Invention

### Problems to be Solved by the Invention

The present invention has been made in an effort to solve the problems of the prior art, and one aspect of the present invention is to provide a technique for sequential immunoaffinity isolation under mild conditions in which upon diagnosis of a specific disease such as cancer, degenerative disease, heart disease or infectious disease, a subpopulation, a sub-subpopulation or a subset thereof containing exosomes associated with the specific disease in a body fluid is separated and recovered intact in high yield and is used as a liquid biopsy sample.

A further aspect of the present invention is to provide a technique for analyzing a liquid biopsy sample containing a subpopulation, a sub-subpopulation or a subset thereof containing target exosomes to discover biomarkers such as specific disease-associated proteins and nucleic acids present in the target exosomes.

### Means for Solving the Problems

A device for preparing an exosome liquid biopsy sample according to an embodiment of the present invention includes solid-state immobilization members, first capture materials specifically binding with first separation markers of first target exosomes associated with a predetermined disease in a population sample containing exosomes released from cells to capture the first target exosomes, and first reversible linkers through which the first capture materials detachably bind to the immobilization members.

A method for preparing an exosome liquid biopsy sample according to an embodiment of the present invention includes (a) allowing first capture materials specifically binding with first separation markers of first target exosomes associated with a predetermined disease to bind to solid-state immobilization members through first reversible linkers, (b) allowing a population sample containing exosomes released from cells to react with the first capture materials bound to the immobilization members such that the first capture materials capture the first target exosomes to separate a subpopulation containing the first target exosomes, and (c) dissociating the first reversible linkers such that the captured first target exosomes are separated from the immobilization members to recover the exosome subpopulation.

A method for analyzing exosome liquid biopsy samples according to an embodiment of the present invention includes (a) preparing exosome liquid biopsy samples containing target exosomes associated with a predetermined disease from exosome population samples containing exosomes released from cells in normal and patient groups, (b) measuring quantitative signals from a plurality of biomarkers of the target exosomes present in the exosome liquid biopsy samples, and (c) analyzing the exosome liquid biopsy samples from the normal and patient groups according to correlations between the plurality of biomarkers based on the measured quantitative signal values.

### Effects of the Invention

According to the present invention, a subpopulation of exosomes having exosome-specific markers (primary separation markers) can be separated from a population containing exosomes relatively less associated with a target disease and the specific exosomes highly associated with the target disease can be enriched and recovered for subsequent isolation. A sub-subpopulation of exosomes having exosome-specific markers (secondary separation markers) in the primary separation solution can be separated from a population and the exosomes containing biomarkers involved in the progression of the target disease can be enriched to provide a liquid biopsy sample.

In addition, a subpopulation, a sub-subpopulation or a subset thereof containing exosomes associated with a specific disease can be separated and recovered from a population sample containing exosomes released from cells in the body to prepare a liquid biopsy sample. The liquid biopsy sample can be analyzed by biomarker profiling to discover biomarkers (e.g., proteins and nucleic acids) specific to the target disease.

Furthermore, since the liquid biopsy sample separated and concentrated from the exosome subpopulation, sub-subpopulation or subset thereof is used as an analyte, non-specific exosomes are removed so that noise can be minimized during analysis and specific exosome signals can be maximized. The use of such exosomes as biomarkers for a specific disease such as cancer minimizes the interference of non-specific exosomes resulting from the heterogeneity of exosomes, which is a current problem, enabling accurate recognition of information on the physiological properties and lineage of parent cells from the liquid biopsy sample.

Moreover, the present invention can be used to diagnose a specific disease such as a cancer disease, degenerative disease, heart disease or infectious disease with significantly high accuracy and is thus effective in early clinical diagnosis of the disease. The present invention enables acquisition of information on the progression or decline of a target disease depending on a therapeutic agent for the disease. Therefore, the present invention can also be applied to companion diagnostics for personalized treatment.

### Brief Description of the Drawings

Fig. 1 is a schematic diagram comparing a conventional process for analyzing biomarkers in a liquid biopsy sample (left) with a process for analyzing biomarkers in a liquid biopsy sample prepared by the present invention (right).
Fig. 2 schematically illustrates the construction of a device for preparing an exosome liquid biopsy sample according to an embodiment of the present invention.
Figs. 3 to 6 schematically illustrate the structures of various embodiments of the first reversible linkers and the second reversible linkers illustrated in Fig. 2.
Figs. 7 to 13 are flowcharts illustrating methods for preparing exosome liquid biopsy samples according to exemplary embodiments of the present invention.
Fig. 14 is a flowchart illustrating a method for analyzing exosome liquid biopsy samples according to an embodiment of the present invention.
Fig. 15 shows the results of immunoaffinity chromatography for CD63-positive (CD63+) exosomes in a sample based on switch-like binding.
Fig. 16 shows the reproducibility of exosome isolation when the immunoaffinity chromatography of Fig. 15 was repeated.
Fig. 17 shows the isolation and enrichment of CD63+ exosomes in a sample by immunomagnetic isolation based on switch-like binding.
Fig. 18 shows the reproducibility of exosome isolation when the immunomagnetic isolation of Fig. 17 was repeated.
Fig. 19 shows the results of physical characterization for CD63+ exosomes isolated based on switch-like binding.
Fig. 20 shows the results of immunochemical characterization for exosome surface markers of CD63+ exosome samples separated based on switch-like binding.
Fig. 21 shows the corrected concentrations of Caveolin 1 (Cavl) markers based on the concentration of CD9 to compensate for variations in concentration between exosome standard samples.
Fig. 22 shows the potential effects of the use of CD63+ exosomes isolated based on switch-like binding as analytes on melanoma cancer diagnosis.
Fig. 23 compares the abilities of affinity isolation systems based on switch-like binding to distinguish melanoma cancer samples from normal samples based on a Cav1/CD9 concentration ratio determined by immunoassay of the separated samples with those of currently commercially available exosome isolation systems.

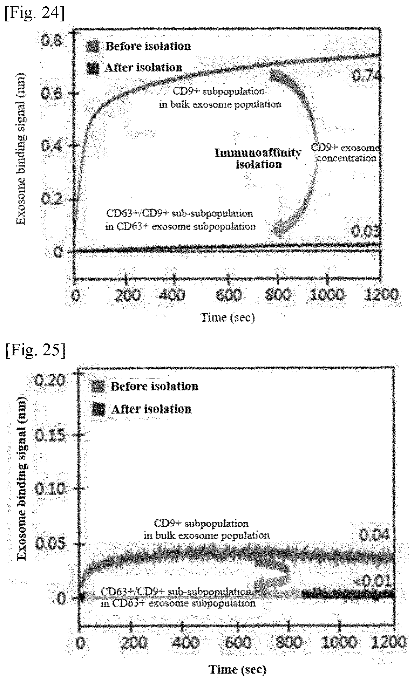

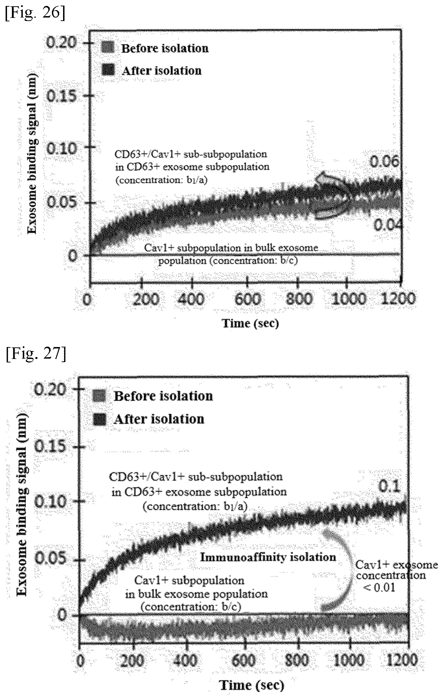

Figs. 24 to 27 show the experimental results obtained when CD9 and Cavl markers in samples before and after immunoaffinity isolation of exosome standard samples were simultaneously measured using label-free sensors in order to verify the effect of exosome subpopulation separation. Specifically, Fig. 24 shows the concentrations of CD9 in a normal exosome sample before and after separation, Fig. 25 shows the concentrations of Cavl in a normal exosome sample before and after separation, Fig. 26 shows the concentrations of CD9 in a cancer cell exosome sample before and after separation, Fig. 27 shows the concentrations of Cavl in a cancer cell exosome sample before and after separation.
Fig. 28 compares correlations between markers for tumor progression, promotion and metastasis based on a marker for tumor suppression after profiling of exosome surface markers for exosome subpopulations in exosome bulk population samples from normal and malignant melanoma patient groups.
Fig. 29 compares correlations between markers for tumor progression, promotion and metastasis based on a marker for tumor suppression after profiling of exosome surface markers for exosome sub-subpopulations in exosome subpopulations separated from exosome bulk populations from normal and malignant melanoma patient groups as samples.
Fig. 30 compares correlations between markers for tumor progression, promotion and metastasis after profiling of exosome surface markers for exosome subpopulations in exosome bulk population samples from normal and malignant melanoma patient groups.
Fig. 31 compares correlations between markers for tumor progression, promotion and metastasis after profiling of exosome surface markers for exosome sub-subpopulations in exosome subpopulation samples separated from exosome bulk populations from normal and malignant melanoma patient groups.
Fig. 32 shows the results of biomarker profiling for exosome subpopulations in exosome bulk population samples from normal and malignant melanoma patient groups when newly defined parameters were applied to the biomarker profiling.
Fig. 33 shows the results of biomarker profiling for exosome subpopulation samples separated from exosome bulk populations from normal and malignant melanoma patient groups when newly defined parameters were applied to the biomarker profiling.
Fig. 34 shows the overall results of two-dimensional parameter analysis for the results of biomarker profiling for exosome subpopulations in exosome bulk population samples (A) from normal and malignant melanoma patient groups and for exosome sub-subpopulations in exosome subpopulation samples (B) separated from the exosome bulk populations when newly defined parameters were applied to the biomarker profiling.
Fig. 35 shows the overall results of two-dimensional parameter analysis for the results of biomarker profiling for sub-subpopulations separated from exosome subpopulation samples from normal and malignant melanoma patient groups when newly defined parameters were applied to the biomarker profiling.
Fig. 36 shows the overall results of two-dimensional parameter analysis for the results of biomarker profiling for exosome sub-subpopulations in exosome subpopulation samples from normal and prostate cancer patient groups when newly defined parameters were applied to the biomarker profiling.
Fig. 37 shows the analytical results for prostate cancer miRNA biomarkers present in exosome population, CD63+ subpopulation, and CD63+/CD81+ sub-subpopulation samples from cultures of prostate cancer-derived cell lines.

### Best Mode for Carrying out the Invention

The objects, specific advantages, and novel features of the present invention will become more apparent from the following detailed description and preferred embodiments, examples of which are illustrated in the accompanying drawings. The same reference numerals and signs denote the same or like components even when they are shown in different accompanying drawings from one another. Although the terms "first", "second", etc. are used herein to describe various components, these components should not be limited by these terms. These terms are only used to distinguish one component from another component. Lest it should obscure the subject matter of the invention, a detailed description of well-known technologies is avoided.

Preferred embodiments of the present invention will now be described in detail with reference to the accompanying drawings.

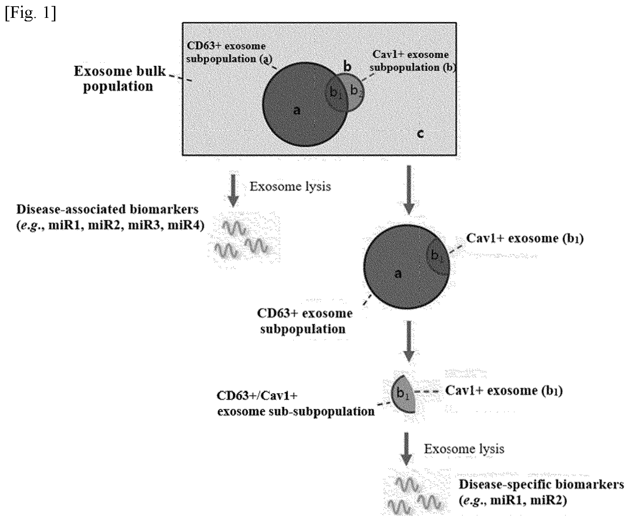

Fig. 1 is a schematic diagram comparing a conventional process for analyzing biomarkers in a liquid biopsy sample (left) with a process for analyzing biomarkers in a liquid biopsy sample prepared by the present invention (right).

The present invention is directed to a technology in which a liquid biopsy sample is prepared by separating exosomes in a body fluid into a subpopulation and subsets thereof, biomarker profiling is performed on the exosome subpopulation and subsets thereof to discover specific disease-associated biomarkers, and the specific disease-associated biomarkers are applied to clinical early diagnosis.

Liquid biopsy is known as a diagnostic method for early determination of the occurrence of a specific disease by extracting a body fluid such as blood, saliva or urine and analyzing exosomes released from specific disease-associated cells. Technologies using exosomes extracted from body fluids as new diagnostic biomarkers for specific diseases such as cancer diseases, degenerative diseases, infectious diseases, and heart diseases have recently been investigated. However, exosomes derived from all cells exist in a mixed state in body fluids, there is a limitation in accurately diagnosing specific diseases such as cancer from a bulk population of exosome samples separated from body fluids, as shown at the left of Fig. 1. It is quite difficult to find specific biomarker molecules present in target exosomes from body fluids as samples. The availability of exosome nanovesicles as biomarkers relies on the enrichment of highly selected markers during exosome isolation. Otherwise, the target exosomes constitute only a very small proportion of the total proteome/transcriptome of body fluids. As such, since it is necessary to sort substances (e.g., biomarkers) present in exosomes, the enrichment of diagnostic markers at exosomal sources such as body fluids helps the discovery of relatively lowly expressed biomarkers that normally would go undetected. Thus, there is a need to develop a technique for isolating and enriching target exosomes. Conventional techniques for exosome isolation are based on ultracentrifugation, precipitation separation, size exclusion chromatography or microfluidics. However, these techniques are limited in selectively detecting trace amounts of target exosomes derived from specific cells such as cancer cells because all exosomes present in body fluids are provided in the form of single populations.

Thus, the present invention employs immunoaffinity isolation for specific markers to separate a specific disease-associated exosome subpopulation and its further extended population *(i.e.* sub-subpopulation), based on the finding that protein markers highly correlated with cancer, for example, Caveolin 1 (Cavl) for melanoma cancer, HER-2/neu for gastric cancer, and LICAM for ovarian cancer are present on the surface of exosomes, as shown at the right of Fig. 1. That is, a subpopulation of exosomes having exosomal markers (e.g., CD63 for melanoma cancer) is primarily separated from a population containing exosomes relatively less associated with a target disease and a sub-subpopulation of exosomes having exosome-specific markers (e.g., Cavl for melanoma cancer) in the primary separation solution is detected or separated to prepare a liquid biopsy sample in which exosomes containing biomarkers (e.g., specific miRNAs) involved in the progression of the target disease are enriched.

Exosome profiling can provide personalized information on individual patients and is crucial for accurate prognosis or differentiated diagnosis. Changes of exosome-derived biomarkers may represent responses to abnormal single conditions. Exosomes in body fluids, for example, plasma exosomes, are released from various cellular sources, for example, blood cells and surrounding tissue.

Various proteins are heterogeneously distributed on the surface of exosomes in body fluids for liquid biopsy. Particularly, specific surface proteins associated with a cancer disease are different in their expression levels but are complexly present even in exosomes from healthy subjects. The types and expression levels of surface proteins, for example, cell type-specific molecules such as tetraspanins (e.g., CD9, CD63, CD81, CD82, and CD151) and histocompatibility complex (MHC) class-I and class-II, adhesion molecules, HSP60, and HSP70, were reported to increase or decrease depending on the characteristics of parent cells. For example, CD63, an exosomal surface protein of the tetraspanin family, is a protein present in the lysosomal membrane of exosomes derived from B cells and was reported to be increased in samples from patients with several cancer diseases, including melanoma cancer. Some studies reported that CD63 can be used as an optimal exosomal marker for disease diagnosis because its expression level varies depending on the type of parental cells, compared to CD81 and CD9 of the same family. For accurate quantitative analysis of exosomes surface proteins, invariant housekeeping markers independent of the characteristics of parent cells can be quantified for normalization. For this normalization, CD9 is predicted to be more suitable than CD63 in the tetraspanin family.

Under such circumstances, immunoaffinity isolation into exosome subpopulations based on exosomal surface proteins can offer the following advantages. First, immunoaffinity isolation based on antigen-antibody binding minimizes the presence of impurities other than target exosomes in solutions after isolation, unlike isolation methods on the basis of density, size, precipitation, etc. As described above, sample matrix effects such as non-specific interactions decrease with decreasing amount of impurities in samples for diagnosis, resulting in improvements of diagnostic sensitivity and specificity. Second, immunoaffinity isolation on a population containing exosomes relatively less associated with a target disease can increase in the concentration of specific disease-associated exosomes in a subsequent subpopulation. This approach is useful when the initial concentration of disease-associated exosomes in the exosome population is low enough to be difficult to detect in a body fluid. In this case, the use of the subpopulation as a sample for diagnosis of a specific disease can lead to an improvement in sensitivity.

The exosome subpopulation can be further separated into a sub-subpopulation of exosomes containing high concentrations of specific disease markers, for example, mRNAs and miRNAs, via a specific exosomal surface protein associated with a specific disease such as cancer. The exosome sub-subpopulation can be provided as a diagnostic sample. Particularly, exosomes released from cancer cells are known to contain various types of RNAs that create local environments such as angiogenesis in the pre-metastatic stage of cancer and play a dominant role in the metastatic stage. Such biomarkers were reported to be loaded in specific exosomes.

In practice, an increased number of exosome-derived proteins have been identified as potential biomarkers for various diseases such as hepatitis, hepatic diseases, renal diseases, and degenerative diseases as well as cancer diseases. Exosomal proteins were reported to be associated with specific diseases, offering the possibility of extending their use as diagnostic or separation targets in the future development of products. For example, CD81 in plasma is an exosomal protein marker associated with chronic hepatitis C, CD63, caveolin-1, TYRP2, VLA-4, HSP70, and HSP90 in plasma are markers associated with melanoma cancer, survivin in plasma is a marker associated with prostate cancer, and L1CAM, CD24, ADAM10, EMMPRIN, and claudin in plasma are markers associated with ovarian cancer. Fetuin-A and ATF 3 in urine are exosomal protein markers associated with acute kidney injury, CD26, CD81, S1c3A1, and CD10 in urine are markers associated with hepatic injury, EGF, resistin, and retinoic acid-induced protein 3 in urine are markers associated with bladder cancer, and PSA and PCA3 in urine are markers associated with prostate cancer. Amyloid peptides in brain tissue are exosomal peptide markers associated with Alzheimer's disease and Tau phosphorylated Thr181 in cerebrospinal fluid is a marker associated with Alzheimer's disease.

Tetraspanins are a family of skeletal membrane proteins that are abundantly found in exosomes. For instance, plasma CD63 positive (CD63+) exosomes are significantly increased in melanoma cancer patient groups compared to in healthy controls. CD81 is another exosomal marker of the tetraspanin family and plays a crucial role in hepatitis C virus binding and/or endocytosis. In addition, the concentration of CD81 in serum exosomes is elevated in patients with chronic hepatitis C and is also associated with the exacerbation of inflammation and fibrosis, suggesting that CD81 in exosomes would be a potential marker for hepatitis C diagnosis and therapeutic response. Moreover, many protein biomarkers in exosomes were reported to be potentially useful for the diagnosis of central nervous system diseases. Glioblastoma-specific epidermal growth factor receptor vIII (EGFRvIII) found in serum exosomes from patients with glioblastoma is useful for the diagnostic detection of glioblastoma. It has also been reported that exosomal amyloid peptides accumulate in brain plaques of patients with Alzheimer's disease. Tau protein phosphorylated at Thr-181 is a well-established biomarker for Alzheimer's disease and is present at an increased concentration in exosomes isolated from cerebrospinal fluid samples of patients with mild Alzheimer's. Based on these findings, the potential value of exosomes has emerged in the early diagnosis of Alzheimer's disease. There is at present no diagnostic test method for identifying early Alzheimer's disease.

Particularly, urinary tract diseases are being explored to determine the potential diagnostic usefulness of proteins in urinary exosomes obtained by non-invasive means. For instance, urinary exosomal fetuin-A is increased in patients with acute kidney injury in intensive care units compared to in healthy subjects. ATF3 is found only in exosomes isolated from patients with acute kidney injury, not in patients with chronic kidney diseases and healthy individuals. Furthermore, urinary exosomal proteins are being investigated for their use as potential biomarkers for bladder cancer and prostate cancer. These investigations revealed that two types of biomarkers for prostate cancer, PCA-3 and PSA, are present in exosomes isolated from the urine of patients with prostate cancer. Overall, exosomal proteins identified in other body fluids are expected to be useful as target platforms in the future development of diagnostic assay systems.

Protein markers highly correlated with cancer, for example, Caveolin 1 (Cavl) for melanoma cancer, HER-2/neu for gastric cancer, and L1CAM for ovarian cancer, were also reported to be present on the surface of exosomes. However, since the contents of these specific exosomes, particularly in samples for early diagnosis, are very low, the introduction of the above-described sequential repeated isolation processes is inevitable for selective isolation of exosomes.

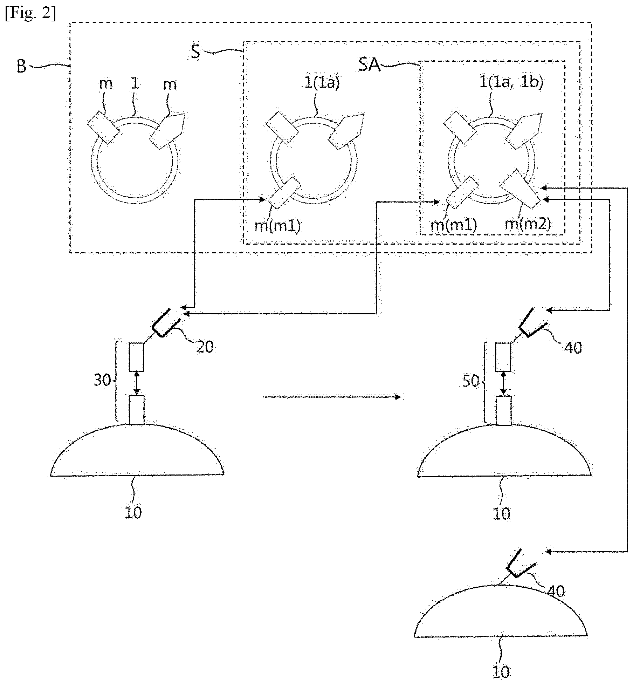

Fig. 2 schematically illustrates the construction of a device for preparing an exosome liquid biopsy sample according to an embodiment of the present invention.

As illustrated in Fig. 2, the device includes solid-state immobilization members 10, first capture materials 20 specifically binding with first separation markers m1 of exosomes 1a associated with a predetermined disease in exosomes 1 released from cells to capture the disease-associated exosomes 1a, and first reversible linkers 30 through which the first capture materials 20 detachably bind to the immobilization members 10.

The device may further include second capture materials 40 binding to the immobilization members 10 to which the first capture materials 20 are not bound and specifically binding with second separation markers m2 of target exosomes 1b in an exosome subpopulation S, which is a population of the disease-associated exosomes 1a, to capture the target exosomes 1b.

The device may further include second reversible linkers 50 through which the second capture materials 40 bind to the immobilization members 10.

The immobilization members 10 are solid-state members. The first reversible linkers 30, the second capture materials 40, and the second reversible linkers 50 can selectively bind to and be immobilized on the surface of the immobilization members 10. After the first reversible linker 30 bound to and immobilized onto one immobilization member 10 unbinds from the immobilization member 10, the second capture material 40 or the second reversible linker 50 may bind to and immobilized onto the immobilization member 10. Alternatively, the first reversible linkers 30 and the second capture materials 40 or the second reversible linkers 50 may be immobilized onto different immobilization members. The immobilization members 10 may be selected from the group consisting of, but not necessarily limited to, hydrogels, magnetic beads, latex beads, glass beads, nanometal structures, porous membranes, nonporous membranes, and combinations thereof. However, any material that can bind with the first reversible linkers 30, the second capture materials 40 or the second reversible linkers 50 may be used without particular limitation for the immobilization members 10.

The first capture materials 20 and the second capture materials 40 are materials capable of selectively capturing predetermined exosomes 1. The first capture materials 20 and the second capture materials 40 specifically bind with the separation markers m of the exosomes 1 to capture the exosomes 1. Here, the separation markers m are biomarkers that are present in a relatively increased amount in a body fluid of a patient with a specific disease compared to in a body fluid a healthy subject. The separation markers may include proteins, nucleic acids, lipids, sugars, peptides, and other biochemical components present on the surface of the exosomes 1. The separation markers m may bind specifically to the first capture materials 20 and the second capture materials 40 through immunochemical interactions such as antigen-antibody reactions. For example, the first capture materials 20 and the second capture materials 40 may be predetermined antibodies reacting with surface proteins of the exosomes 1.

The first separation markers m1 specifically binding to the first capture materials 20 are present on the surface of the exosomes 1a associated with a predetermined disease. Accordingly, the disease-associated exosomes 1a having the first separation markers m1 captured by the first capture materials 20 can be isolated from a bulk population B of the exosomes 1 released from cells. Here, a population of the isolated disease-associated exosomes 1a becomes the subpopulation S. The second separation markers m2 of the target exosomes 1b among the disease-associated exosomes 1a in the exosome subpopulation S react specifically with and are captured by the second capture materials 40, with the result that an exosome sub-subpopulation SA is separated from the exosome subpopulation S. The first separation markers m1 on the exosomes 1 exhibit a relatively low accuracy compared to the second separation markers m2 when used to diagnose a specific disease. However, the first separation markers m1 are present at a relatively high concentration in body fluids and may have more comprehensive characteristics because they are located at a higher level on a system classification based on the amount of markers in exosome populations.

The present invention may use an exosome liquid biopsy sample that is prepared by primarily separating a subpopulation S of surface protein marker (first separation marker) positive exosomes from an exosome population including disease-specific markers, whose concentration in a body fluid is predicted to be low, and enriching and recovering the exosomes. Alternatively, the present invention may use an exosome liquid biopsy sample that is prepared by secondarily separating and concentrating the disease-specific markers (second separation markers) from the subpopulation S to recover an exosome sub-subpopulation S. In the present invention, a subpopulation S of surface protein marker (first separation marker) positive exosomes is primarily separated from an exosome population including disease-specific markers, whose concentration in a body fluid is predicted to be low, the exosomes are enriched and recovered, and the disease-specific markers (second separation markers) are secondarily separated and concentrated from the subpopulation S to recover an exosome sub-subpopulation S. Here, the separation, concentration, and recovery processes on other exosomal surface protein markers can be repeated twice or more according to the purpose. A subset recovered by separation and concentration from the exosome sub-subpopulation SA may be used as an exosome liquid biopsy sample.

The conversion of exosome samples in the form of bulk populations prepared by simply concentrating body fluids to disease-relevant exosome samples selectively separated from body fluids can lead to an improvement in the diagnostic accuracy of liquid biopsy. Caveolin 1 (Cavl), an exosomal surface protein, is significantly increased in the blood of patients with melanoma cancer, which is a skin cancer that begins in the melanocytes, compared to in the blood of healthy subjects, with the result that the Cav1- positive (Cav1+) exosome-based diagnostic sensitivity is 68%, which is higher than the diagnostic sensitivity (43%) based on CD63 as another melanoma cancer marker. The clinical use of liquid biopsy with improved performance requires selective isolation and enrichment of target exosomes such as Cav1+ exosomes but is difficult to achieve by general processes known in the art because of a very low proportion of the target exosomes in all exosomes from body fluids.

Therefore, the present invention intends to provide a method for diagnosing melanoma cancer with high accuracy and sensitivity using, as a test sample, a subpopulation or sub-subpopulation containing Cav1+ exosomes selectively isolating from a body fluid. In consideration of the yield and reproducibility of exosome isolation, Cav1+ exosomes are not used as isolation targets but a population of exosomes associated with carcinogenesis is used as a primary separation target. For example, since CD63 protein, a house-keeping protein abundantly found in general exosomes, is increased and expressed on the surface of exosomes when several cancers (including melanoma cancer) occur, compared to other surface proteins, this surface protein can be used as a separation marker m1 to separate a CD63+ exosome subpopulation S and Cavl can be used as a second separation marker m2 to separate a CD63+/Cav1+ exosome sub-subpopulation SA from the CD63+ exosome subpopulation S.

The first capture materials 20 can be immobilized onto and separated from the immobilization members 10 via the first reversible linkers 30. The second capture materials 40 may directly bind to and immobilized onto the immobilization members 10 without being separated from the immobilization members 10. Alternatively, the second capture materials 40 may be immobilized onto and separated from the immobilization members 10 through the second reversible linkers 50 introduced therebetween.

The first capture materials 20 detachably bind to the immobilization members 10 through the first reversible linkers 30 and the second capture materials 40 detachably bind to the immobilization members 10 through the second reversible linkers 50. Here, when the first capture materials 20 do not bind to the immobilization members 10 when the second capture materials 40 are immobilized onto the immobilization members 10. That is, the second capture materials 40 are immobilized onto the immobilization members 10 through the second reversible linkers 50 when the first capture materials 20 do not bind to the immobilization members 10. The first reversible linkers 30 and the second reversible linkers 50 may be composed of the same or different materials and have the same or different structures. The materials and structures for the first reversible linkers 30 and the second reversible linkers 50 will be described in detail below.

Exosome immunoaffinity isolation under mild conditions is possible using the reversible linkers based on ligand-binder binding. The reversible linkers initially serve to immobilize capture materials (antibodies) capable of capturing specific exosomes 1 onto the immobilization members 10 and are dissociated by varying reaction conditions after capture of the exosomes 1 on the immobilization members 10 so that the captured exosomes 1 can be recovered. For example, the reversible linkers may be used based on various reactions, including sugar-sugar receptor binding through hydrophilic and hydrophobic binding, ion-ion or ion-ion receptor binding, substrate-enzyme reaction, antigen-antibody reaction, nucleic acid hybridization, hormone-cell receptor reaction, and ligand-nanostructure reaction, and particularly binding reactions with polyhistidine-tag, biotin-avidin linkage, and chelators. The reversible linkers can be dissociated by changing the structure of the binders after binding or controlling the binder-ligand affinity of the linkers, such as competitive reactions for the binders. Therefore, the use of the reversible linkers enables the isolation and recovery of the target exosomes 1b under mild conditions after immunological capture of the exosomes 1 in the sample, avoiding the need for harsh conditions such as acidic pH.

Immunoaffinity isolation using the binding reaction-based reversible linkers may be any process that enables isolation and recovery based on physical, biological, and chemical properties such as density, size, solubility, membrane permeability, molecular structure, and magnetism. Particularly, the use of a specially designed immunoaffinity isolation process such as chromatography or magnetic isolation enables isolation and recovery of a trace amount of exosomes in body fluids through control over binder-ligand affinity in the linkers. For example, the use of "switch-like binding" of a recognition material recognizing a change in the structure of binders in the linkers caused during ligand binding enables isolation and recovery of exosomes by immunoisolation only depending on the presence or absence of ligands. The introduction of competitive binding reactions using ligand-like structures competing with ligands for binders in ligand-binder reactions during immunoisolation enables isolation and recovery of exosomes depending on the presence or absence of competitive binding reactions.

This immunoisolation process may be performed based on reversible chemical crosslinking-decrosslinking as well as reversible binding such as ligand-binder binding. Particularly, crosslinking is performed through a chemical reaction between polymers and decrosslinking is then performed under certain conditions or by cleaving the chemically crosslinked bonds through light energy.

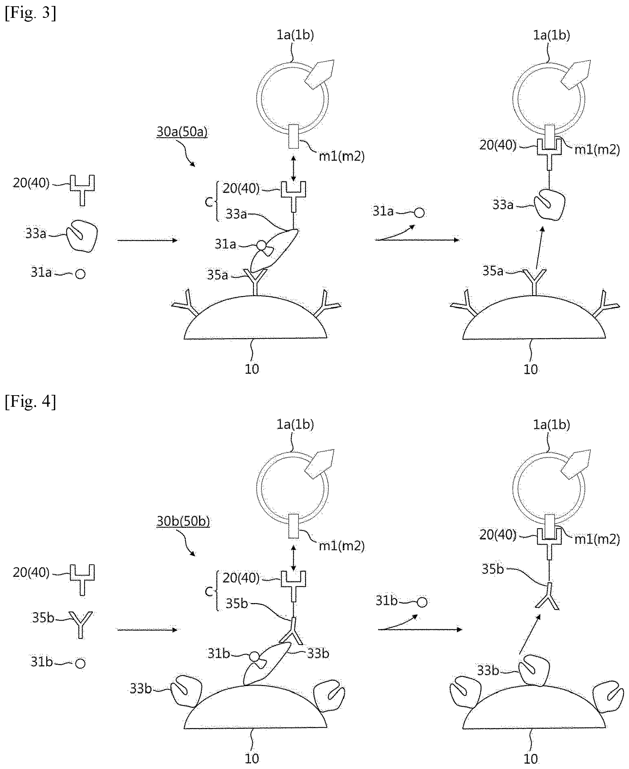

A detailed description will be given of the first reversible linkers 30 and the second reversible linkers 50. The reversible linkers may be formed according to first to fourth embodiments that follow. Specifically, the first reversible linkers 30 and 30a-30d and the second reversible linkers 50 and 50a-50d may be formed according to one of the first to fourth embodiments. Figs. 3 to 6 schematically illustrate the structures of various embodiments of the first reversible linkers and the second reversible linkers illustrated in Fig. 2.

As illustrated in Fig. 3, each of the reversible linkers 30a and 50a according to a first embodiment of the present invention includes a ligand 31a, a binder 33a, and a recognition material 35a.

The ligand 31a is a material capable of detachably binding to the binder 33a and may be selected from the group consisting of sugar molecules, ions, substrates, antigens, peptides, vitamins, growth factors, hormones, and combinations thereof.

The binder 33a is a material whose conformation is changed when bound with the ligand 31a. The binder 33a may be selected from the group consisting of sugar-binding proteins, ion-binding proteins, enzymes, antibodies, avidins, aptamers, cell receptors, nanostructures, and combinations thereof. Accordingly, the binder 33a and the ligand 31a can form a binder-ligand pair such as a sugar-binding protein-sugar molecule pair, an ion-binding protein-ion pair, a biotin-avidin pair, an enzyme-substrate pair, an antigen-antibody pair, an aptamer-ligand pair, a cell receptor-ligand pair or a nanostructure-ligand pair. The most representative examples of the binder 33a and the ligand 31a include calcium-binding proteins (CBPs) and calcium ions, respectively. However, the binder 33a and the ligand 31a are not necessarily limited to the above-mentioned materials and may be any materials that can detachably bind to each other to cause their conformational changes. In the reversible linker, the binder 33a is conjugated with a first capture material 20 and/or a second capture material 40 to form a binder-capture material conjugate C.

The recognition material 35a is a material that specifically binds to the binder 33a whose conformation has been changed by binding with the ligand 31a. The recognition material 35a may be selected from the group consisting of antibodies, protein receptors, cell receptors, aptamers, enzymes, nanoparticles, nanostructures, heavy metal chelators, and combinations thereof. However, these materials are merely illustrative and the scope of the present invention is not necessarily limited thereto. For example, a calcium ion as the ligand 31a primarily binds to a calcium binding protein as the binder 33a to cause a conformational change of the calcium binding protein and the calcium binding protein whose conformation has been changed reacts with and binds to an antibody as the recognition material 35a ("antigen-antibody reaction"). In the reversible linker, the recognition material 35a is immobilized on the surface of an immobilization member 10.

After an exosome 1 is captured by the first capture material 20 and/or the second capture material 40 bound to the immobilization member 10 via the reversible linker 30 and/or 50, the captured exosome 1a or 1b can be recovered by varying the reaction conditions to dissociate the reversible linker. The reversible linker 30a or 50a can be dissociated when the binder 33a whose conformation has been changed is restored to its original conformation. For example, after the antibody (recognition material) binds with the calcium-binding protein (binder) whose conformation has been changed by binding with the calcium ion (ligand) and captures the exosome 1, a calcium ion-free recovery solution is added to detach the calcium ion from the calcium-binding protein, with the result that the conformation of the calcium-binding protein can be restored and the calcium-binding protein can be separated from the antibody.

Referring to Fig. 4, each of the reversible linkers 30b and 50b according to a second embodiment of the present invention includes a ligand 31b, a binder 33b, and a recognition material 35b.

The ligand 31b, the binder 33b, and the recognition material 35b of the reversible linker 30b or 50b according to the second embodiment correspond to the ligand 31a, the binder 33a, and the recognition material 35a of the reversible linker 30a or 50a according to the first embodiment, respectively, and only the differences will be mainly described below.

In the reversible linker 30a or 50a according to the first embodiment, the binder 33a is conjugated with the capture material 20 or 40 to form the polymer C and the recognition material 35a is immobilized on the surface of the immobilization member 10. In contrast, in the reversible linker 30b or 50b according to the second embodiment, the binder 33b is immobilized on the surface of the immobilization member 10 and the recognition material 35b is conjugated with a capture material 20 or 40 to form a recognition material-capture material polymer C. Aside from these, the reversible linker 30b or 50b is the same as the reversible linker 30a or 50a according to the first embodiment. Thus, a detailed description of the reversible linker 30b or 50b is omitted.

As an example of each of the reversible linkers 30 and 50 according to the first and second embodiments that enables the recovery of an exosome subpopulation or sub-subpopulation in a sample under mild conditions, an antibody may be used as the recognition material 35a or 35b that specifically recognizes a conformational change caused when a calcium binding protein as the binder 33a or 33b binds and reacts specifically with a calcium ion as the ligand 31a or 31b. The binder-recognition material reaction may be an antigen-antibody reaction in which the binder quickly binds to or unbinds from the recognition material depending on the presence or absence of the ligand 31a or 31b such as a calcium ion. This binding or unbinding is described as "switch-like reversible binding" because its principle is similar to that of light turning on/off by switching.

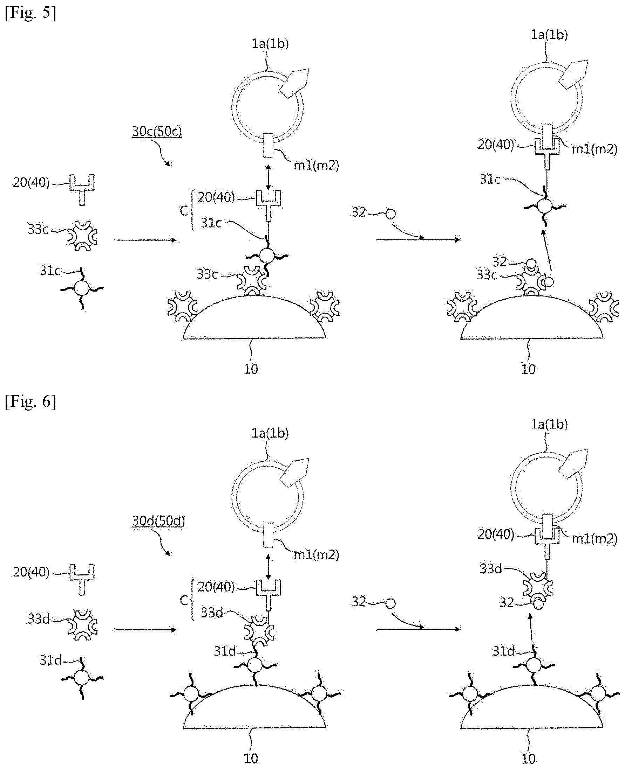

As illustrated in Fig. 5, each of the reversible linkers 30c and 50c according to a third embodiment of the present invention includes a ligand 31c and a binder 33c.

The ligand 31c of the reversible linker 30c or 50c according to the third embodiment is conjugated with a first capture material 20 and/or a second capture material 40 to form a capture material-ligand conjugate C.

The binder 33c of the reversible linker 30c or 50c is immobilized on the surface of the immobilization member 10 and binds with the capture material-ligand conjugate C specifically bound to an exosome 1a or 1b, with the result that the exosome 1a or 1b is captured by the immobilization member 10. The binder 33c bound with the ligand 31c binds with another ligand 32 competing with the ligand 31c. As a result, the ligand 31c is detached and the exosome 1a or 1b can be separated and recovered from the immobilization member 10. Here, the ligand 31c may be selected from the group consisting of polyhistidine-tags (His-tags), vitamins such as biotin, sugar molecules, ions, substrates, antigens, amino acids, peptides, nucleic acids, growth factors, hormones, and combinations thereof. The binder 33c may be selected from the group consisting of divalent metal ions, avidins, sugar-binding proteins, ion-binding proteins, enzymes, antibodies, aptamers, protein receptors, cell receptors, nanostructures, and combinations thereof. The competitive ligand 32 may be selected from the group consisting of imidazole, vitamins such as biotin, sugar molecules, ions, substrates, antigens, amino acids, peptides, nucleic acids, growth factors, hormones, and combinations thereof.

For example, a His-tag, a divalent metal ion, and imidazole may be used as the ligand 31c, the binder 33c, and the competitive ligand 32, respectively. A His-tag is an amino acid motif in proteins that consists of at least six histidine (His) residues and is commonly positioned at the N- or C-terminus of the protein. His-tag is usually used for purification of recombinant proteins after expression. Here, the protein labeled with His-tag is captured based on affinity with a divalent nickel or cobalt ion bound with a chelator on a sepharose/agarose resin and is then recovered by addition of imidazole-containing recovery solution.

Referring to Fig. 6, each of the reversible linkers 30d and 50d according to a fourth embodiment of the present invention includes a ligand 31d and a binder 33d.

The ligand 31d and the binder 33d of the reversible linker 30d or 50d correspond to the ligand 31c and the binder 33c of the reversible linker 30c or 50c according to the third embodiment, respectively, and only the differences will be mainly described below.

In the reversible linker 30c or 50c according to the third embodiment, the ligand 31c is conjugated with a capture material 20 or 40 and the binder 33c is immobilized on the surface of the immobilization member 10. In contrast, in the reversible linker 30d or 50d, the ligand 31d is immobilized on the surface of the immobilization member 10 and the binder 33d is conjugated with the capture material 20 or 40 to form a capture material-binder conjugate C. As in the reversible linker 30c or 50c according to the third embodiment, the ligand 31d binds to the binder 33d and the binder 33d binds with another ligand 32 competing with the ligand 31d. As a result, the ligand 31d is separated from the binder 33d.

The device for preparing an exosome liquid biopsy sample may further include a reactor (not shown). The reactor may accommodate the immobilization members 10 therein. When an exosome sample 1 is added to the reactor, disease-associated exosomes 1a are captured by the immobilization members 10 via the first reversible linkers 30 and the first capture materials 20 in the reactor, and an exosome subpopulation S is separated. The first reversible linkers 30 are dissociated to recover the exosome subpopulation S. The recovered exosome subpopulation S is added as a sample to the reactor. Target exosomes 1b are captured by the immobilization members 10 via the second reversible linkers 50 and the second capture materials 40 and an exosome sub-subpopulation SA is separated. The second reversible linkers 50 are dissociated to recover the exosome sub-subpopulation SA. Here, the immobilization members 10 are concentrated using magnetic force, gravitational force, and/or centrifugal force to concentrate the exosome subpopulation S and/or the exosome sub-subpopulation SA. For example, when magnetic beads are used as the immobilization members 10, a magnet is used to capture the immobilization members 10 bound with the disease-associated exosomes 1a and/or the target exosomes 1b. Then, the supernatant is discarded and, from the magnetic beads, the exosome subpopulation S or the exosome sub-subpopulation SA can be recovered and concentrated.

Hereinafter, a method for preparing a liquid biopsy sample using the device will be described. The device has been described above and a repeated explanation is omitted or simplified.

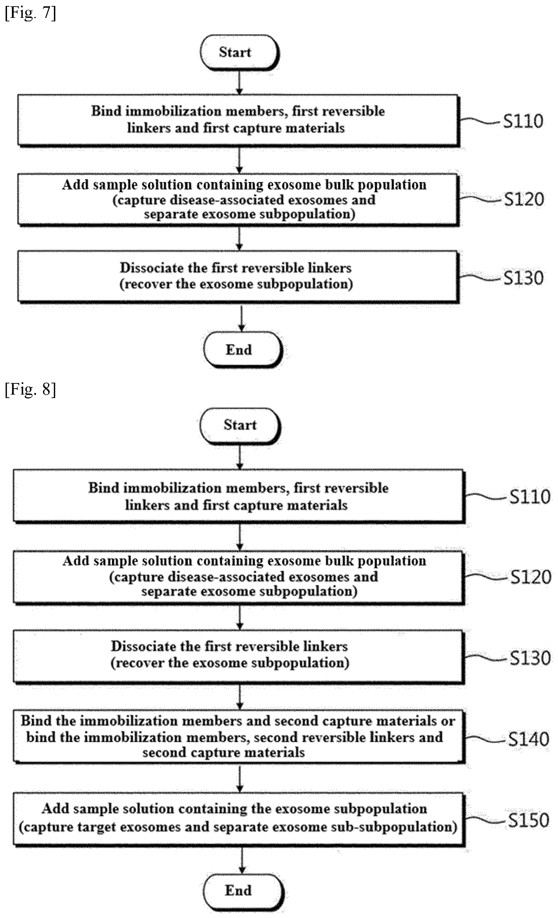

Figs. 7 to 13 are flowcharts illustrating methods for preparing exosome liquid biopsy samples according to exemplary embodiments of the present invention.

Referring to Fig. 7, a method for preparing an exosome liquid biopsy sample according to a first embodiment of the present invention includes (S110) allowing first capture materials specifically binding with first separation markers of exosomes associated with a predetermined disease in exosomes released from cells to bind to solid-state immobilization members through first reversible linkers, (S120) allowing a sample solution containing an exosome population to react with the first capture materials bound to the immobilization members such that the first capture materials capture the disease-associated exosomes to separate a subpopulation containing the disease-associated exosomes, and (S130) dissociating the first reversible linkers such that the captured disease-associated exosomes are separated from the immobilization members to recover the exosome subpopulation.

In the method according to the first embodiment of the present invention, a bulk population of exosomes released from cells or a body fluid is used as a sample solution and an exosome subpopulation primarily separated and recovered from the sample solution can be provided as a liquid biopsy sample.

As illustrated in Fig. 8, the method may further include (S140) allowing second capture materials specifically binding with second separation markers of target exosomes in the exosome subpopulation to bind to the immobilization members and (S150) allowing an exosome subpopulation solution containing the recovered exosome subpopulation to react with the second capture materials bound to the immobilization members such that the second capture materials capture the target exosomes to separate a sub-subpopulation of the target exosomes. Here, the second capture materials may be bound to the immobilization members via second reversible linkers.

First, to separate and recover an exosome subpopulation, first capture materials and first reversible linkers are allowed to react with immobilization members (S110). Here, the first capture materials are bound to the immobilization members via the first reversible linkers.

Next, a sample solution is allowed to react with the first capture materials (S120). Here, the first capture materials bind specifically to first separation markers of disease-associated exosomes present in the sample solution. As a result, a subpopulation containing the disease-associated exosomes is captured by the immobilization members and is separated from the sample solution.

After separation of the exosome subpopulation, the first reversible linkers are dissociated (S 130). At this time, the disease-associated exosomes are separated from the immobilization members to recover the exosome subpopulation.

After the disease-associated exosomes are separated from the immobilization members, the immobilization members may be reused for binding with second capture materials or second capture materials may be allowed to bind to new immobilization members (S140). At this time, the second capture materials may be directly bound to the immobilization members. Alternatively, the second capture materials may be bound to the immobilization members through second reversible linkers.

Next, an exosome subpopulation solution containing the recovered exosome subpopulation is allowed to react with the second capture materials (S 150). At this time, since the second capture materials bind specifically with second separation markers of target exosomes in the exosome subpopulation, the target exosomes are immobilized onto the immobilization members, with the result that a sub-subpopulation of the target exosomes is separated. Instead of recovering the separated exosomes, the exosome subpopulation remaining unrecovered and immobilized onto the immobilization members can be provided as a liquid biopsy sample. That is, diagnostic biomarkers for a specific disease can be directly analyzed without the need to recover the exosomes after separation of the sub-subpopulation. Alternatively, after dissolution of the exosomes, eluted biomarkers can be analyzed. In this case, only biomarkers can be directly used as samples without the need to recover the exosomes from the sub-subpopulation in the final separation step of a process for sequential immunoaffinity isolation.

Hereinafter, the present invention will be described in more detail with reference to specific examples of the first and second reversible linkers.

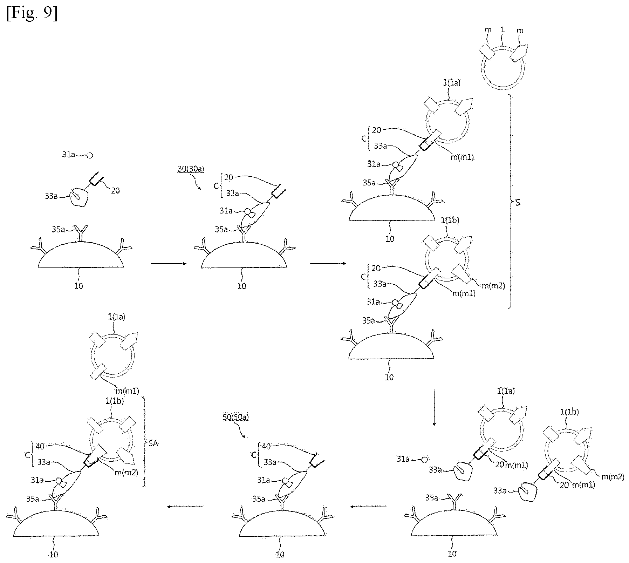

Fig. 9 illustrates a method for preparing an exosome subpopulation as a liquid biopsy sample using the first and second reversible linkers 30a and 50a according to the first embodiment. Referring to Fig. 9, first, recognition materials 35a are immobilized on the surface of immobilization members 10 and a solution containing ligands 31a, binders 33a, and first capture materials 20 are allowed to react with one another. As a result of this reaction, the binders 33a and the first capture materials 20 form binder-first capture material conjugates C and the recognition materials 35a bind with the conjugates C. Here, the ligands 31a present in the ligand solution bind to the binders 33a to cause a conformational change of the binders 33a and the recognition materials 35a specifically recognize and bind with the binders 33a whose conformation has been changed. Next, a sample solution containing a bulk population of exosomes 1 is added. Disease-associated exosomes 1a having first separation markers m1 specifically binding to the first capture materials 20 are captured by the immobilization members 10, so that an exosome subpopulation S is separated. After separation of the exosome subpopulation S, a recovery solution containing the ligands 31a is added. The ligands 31a bound to the binders 33a are detached, and as a result, the changed conformation of the binders 33a is restored to their original state, leading to the unbinding of the binders 33a from the recognition materials 35a. Consequently, the conjugates C bound to the disease-associated exosomes 1a of the exosome subpopulation S are separated from the recognition materials 35a to recover the exosome subpopulation S.

Next, the disease-associated exosomes 1a are separated and the immobilization members 10 immobilized with the recognition materials 35a are reused to allow the second reversible linkers 50a to react with the second capture materials 40. At this time, the second reversible linkers 50a share the recognition material 35a with the first reversible linkers 30a and immobilize the second capture materials 40 instead of the first capture materials 20 onto the immobilization members 10. Then, a solution of the recovered exosome subpopulation S is added. As a result, the second capture materials 40 bind specifically with second separation markers m2 of target exosomes 1b in the solution of the exosome subpopulation S, so that an exosome sub-subpopulation SA is separated. Since the exosome subpopulation S contain the binders 33a of the first reversible linkers 30a, the recognition materials 35a bound to the immobilization members 10 may react with the binders 33a of the first reversible linkers 30a. The reaction can be prevented by blocking the remaining binding sites on the recognition materials 35a bound to the immobilization members 10 with appropriate reactive components (e.g., the binders 33a) after the reaction between the second reversible linkers 50a with the second capture materials 40.

A description will be given of a method for separating a CD63+/Cav1+ exosome sub-subpopulation SA using the first reversible linkers and the second reversible linkers according to the first embodiment of the present invention. Antibodies as the reversible recognition materials are immobilized on the solid surfaces (bead surfaces) and calcium binding proteins (CBPs) as the binders are conjugated with specific antibodies as the first capture materials to CD63 exosomal surface proteins via biotin-streptavidin linkages (CBP-capture antibody conjugates). The CBP-capture antibody conjugates are dissolved in a solution containing calcium ions (*e.g.,* >10 mM Ca²⁺) and are added to the immobilized reversible recognition materials. The conjugates are immobilized on the solid surfaces by a "switch-on" recognition reaction. A bulk exosome sample is prepared and added to the same solution. The exosomes having CD63 markers react with and are captured by the capture antibodies on the solid surfaces. After washing with the same solution, a calcium-free exosome recovery solution is added. The calcium ions are detached from the CBPs, resulting in a "switch-off state. Thus, the captured exosomes bound with the capture antibodies can be recovered into the solution. As a consequence, a subpopulation of the CD63+ exosomes is separated and recovered. The exosomes can be enriched according to the volume ratio between the sample solution and the recovery solution used. In addition, the solid surfaces can be regenerated and reused for the separation of other samples.

For sequential separation of exosome sub-subpopulations, reversible recognition materials are immobilized on the regenerated solid surfaces or new solid surfaces, the specific antibodies to Cavl exosomal surface proteins highly associated with a specific disease are conjugated with the CBPs, and the CBP-capture antibody conjugates are immobilized on the solid surfaces through a "switch-on" recognition reaction as above. While maintaining the same state, the CD63+ exosome subpopulation recovered after the primary separation is added as a secondary separation sample. The CD63 and Cavl marker-containing (CD63+/Cav1+) exosomes react with and are captured by the capture antibodies on the solid surfaces. After washing with the same solution, a calcium-free exosome recovery solution is added. This results in a "switch-off' state, allowing the captured exosomes to be recovered into the solution. As a consequence, a sub-subpopulation of the CD63+/Cav1+ exosomes is secondarily separated and recovered.

When the separated exosome sub-subpopulation does not need to be recovered, the sub-subpopulation can be separated by directly immobilizing the capture antibodies on the solid surfaces without the need to introduce the second reversible linkers. In this case, immunoassay is performed mainly on the captured exosomes. Alternatively, the captured exosomes are directly lysed to extract nucleic acids, which are used for molecular testing.

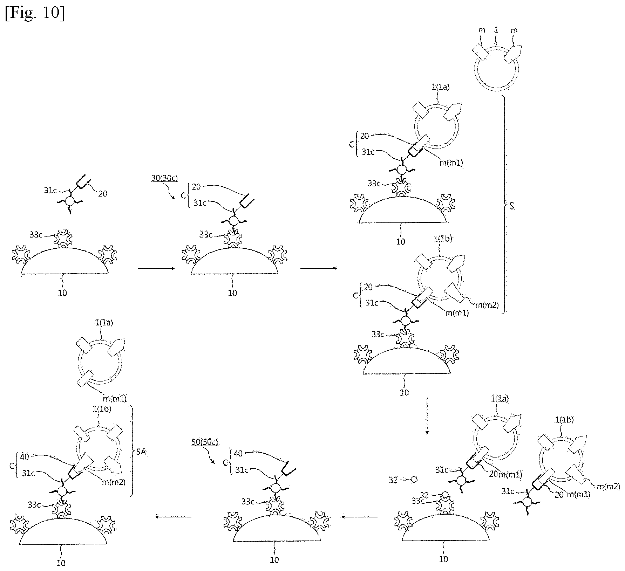

Fig. 10 illustrates a method for preparing an exosome sub-subpopulation as a liquid biopsy sample using the first reversible linkers 30c and the second reversible linkers 50c according to the third embodiment. Referring to Fig. 10, ligands 31c are conjugated with first capture materials 20 specifically reacting with first separation markers m1 of disease-associated exosomes 1a, the ligand-capture material conjugates are immobilized based on affinity with binders 33c bound to the immobilization members 10, an exosome sample is added, an exosome subpopulation S is captured and separated, and a recovery solution containing competitive ligands 32 is used to recover the exosome subpopulation S.

Next, second capture materials 40 specifically reacting with second separation markers m2 of the target exosomes 1b are conjugated with the ligands 31c, the resulting conjugates C are immobilized onto the binders 33c bound to the immobilization members 10, and a solution of the exosome subpopulation S is added to capture and separate an exosome sub-subpopulation SA. Since the exosome subpopulation S contain the ligands 31c of the first reversible linkers 30c, the binders 33c bound to the immobilization members 10 may react with the ligands 31c of the first reversible linkers 30c. The reaction can be prevented by blocking the remaining binding sites on the binders 33c bound to the immobilization members 10 with appropriate reactive components (e.g., the ligands 31c) after the reaction between the second reversible linkers 50c with the second capture materials 40.

A description will be given of a method for separating a CD63+/Cav1+ exosome sub-subpopulation SA using the first reversible linkers and the second reversible linkers according to the third embodiment of the present invention. Nickel ions as the binders are immobilized on the solid surfaces (bead surfaces) and His-tags as the ligands are conjugated with specific antibodies to CD63 exosomal surface proteins as the first separation markers m1. The His-tag-capture antibody conjugates are added to the immobilized nickel ions. The conjugates are immobilized on the solid surfaces by a binder-ligand reaction. A bulk exosome sample is prepared and added to the same solution. The CD63 exosomes react with and are captured by the capture antibodies on the solid surfaces. After washing with the same solution, an exosome recovery solution containing a high concentration of competitive ligands (imidazole) is added. The His-tags bound to the nickel ions are detached by the competitive reaction. Thus, the captured exosomes can be recovered into the solution. As a consequence, a CD63+ exosome subpopulation is separated and recovered and the solid surfaces can be regenerated and reused for next sample separation.

The above isolation process is applied to the separation of a CD63+/Cav1+ exosome sub-subpopulation from the recovered CD63+ exosome subpopulation. In this case, His-tags as the ligands are conjugated with antibodies specific for Cavl exosomal surface proteins as the second separation markers. After the conjugates are immobilized on the solid surfaces through a binder-ligand reaction, the CD63+ exosome subpopulation is added as a sample. The CD63+/Cav1+ exosomes react with and are captured by the capture antibodies on the solid surfaces. After washing, an exosome recovery solution containing a high concentration of competitive ligands (imidazole) is added. the CD63+/Cav1+ exosomes bound to Con A can be recovered into the solution. As a consequence, a sub-subpopulation of the CD63+/Cav1+ exosomes is separated and recovered.

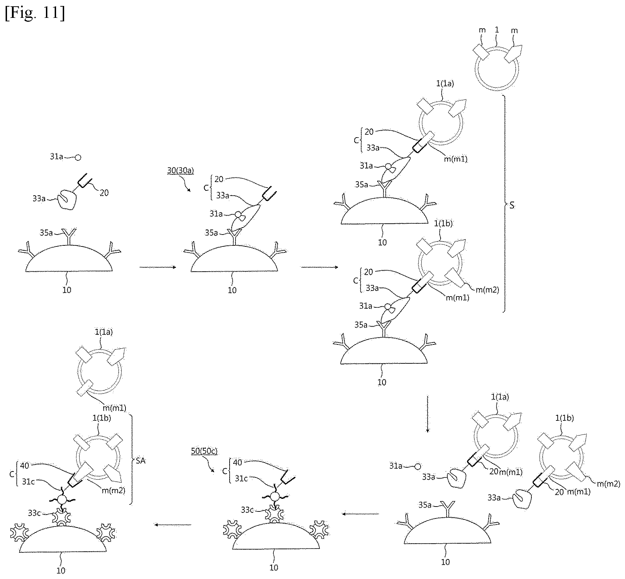

Fig. 11 illustrates a method for preparing an exosome sub-subpopulation as a liquid biopsy sample using the first reversible linkers 30a according to the first embodiment and the second reversible linkers 50c according to the third embodiment. Referring to Fig. 11, an exosome subpopulation S can be separated and recovered using the first reversible linkers 30a according to the first embodiment, as described above, and an exosome sub-subpopulation SA can be separated using the second reversible linkers 50c according to the third embodiment (Fig. 10). The exosome subpopulation S contains the binders 33a of the first reversible linkers 30a, but the binders 33c according to the third embodiment unreactive with the binders 33a according to the first embodiment bind to the immobilization members 10 when the exosome sub-subpopulation SA is separated. That is, the use of the second reversible linkers 50c acting in a mechanism different from that of the first reversible linkers 30a avoids the need for the blocking process described above.

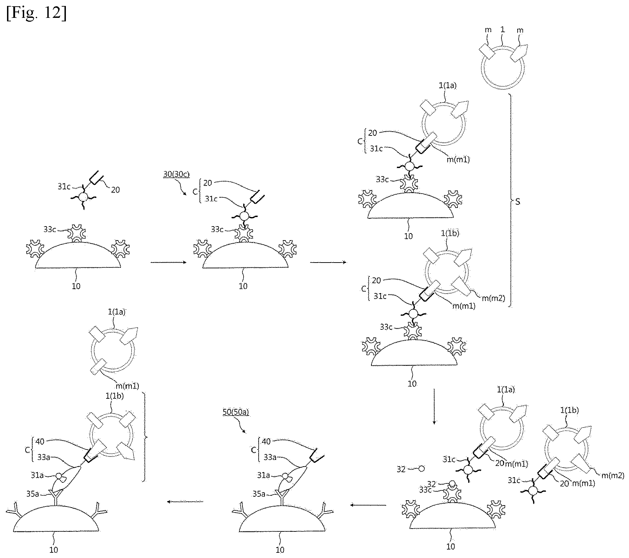

Fig. 12 illustrates a method for preparing an exosome sub-subpopulation as a liquid biopsy sample using the first reversible linkers 30c according to the third embodiment and the second reversible linkers 50a according to the first embodiment. Referring to Fig. 12, an exosome subpopulation S can be separated and recovered using the first reversible linkers 30c according to the third embodiment (Fig. 10) and an exosome sub-subpopulation SA can be separated using the second reversible linkers 50a according to the first embodiment (Fig. 9). The exosome subpopulation S contains the ligands 31c of the first reversible linkers 30c, but the second reversible linkers 50a distinguished from the first reversible linkers 30c are adopted to separate the exosome sub-subpopulation SA, avoiding the need for the blocking process described above.

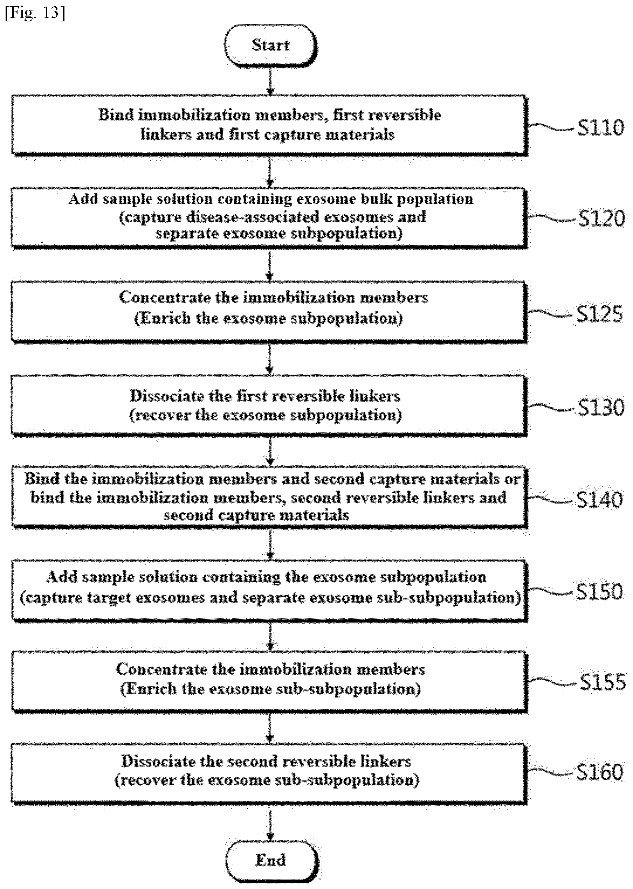

Fig. 13 is a flowchart illustrating a method for preparing an exosome sub-subpopulation as a liquid biopsy sample according to a further embodiment of the present invention.

Referring to Fig. 13, the method may further include (S160) recovering the exosome sub-subpopulation. Here, the second capture materials bind to the immobilization members through the second reversible linkers. Dissociation of the second reversible linkers enables the separation of the captured target exosomes from the immobilization members and the recovery of the exosome sub-subpopulation. The captured target exosomes can be analyzed for biomarkers. Alternatively, after dissolution of the captured target exosomes, eluted biomarkers can be analyzed.

The method may further include (S125) concentrating the exosome subpopulation. The exosome subpopulation may be concentrated after addition of the sample solution and before addition of the recovery solution. Specifically, the immobilization members bound with the disease-associated exosomes of the exosome subpopulation are concentrated using magnetic force, gravitational force, and/or centrifugal force. For example, when magnetic beads are used as the immobilization members 10, a magnetic force is used to capture the immobilization members in predetermined spaces of a mixed solution of the ligand solution and the sample solution. Then, the exosome subpopulation can be concentrated by removing a portion (*e.g.,* supernatant) of the mixed solution where the immobilization members are absent.

In the same manner as the concentration of the exosome subpopulation, the exosome sub-subpopulation can be concentrated by concentrating the immobilization members bound with the target exosomes using magnetic force, gravitational force, and/or centrifugal force (S155).

A description will be given of a method for analyzing exosome liquid biopsy samples prepared by the preparation method.

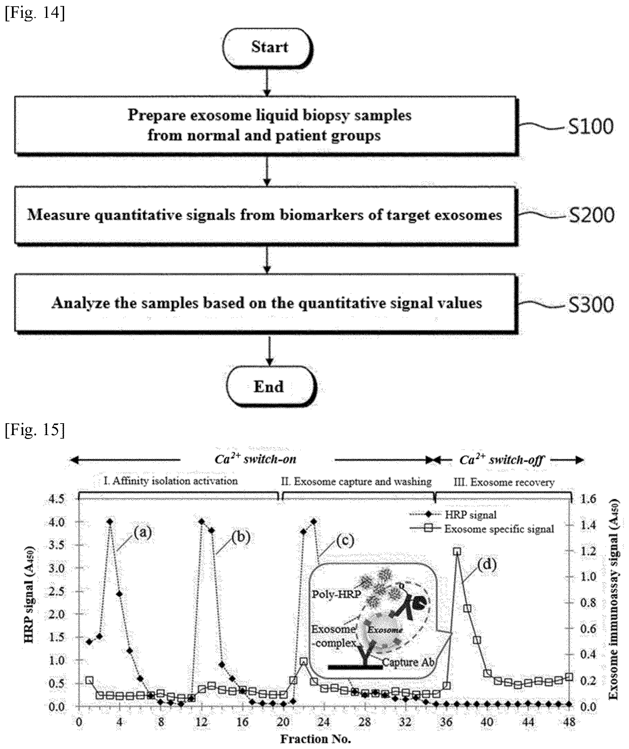

Fig. 14 is a flowchart illustrating a method for analyzing exosome liquid biopsy samples according to an embodiment of the present invention.

As illustrated in Fig. 14, the method includes (S100) capturing target exosomes associated with a predetermined disease from exosome population samples containing exosomes released from cells in normal and patient groups to prepare exosome liquid biopsy samples containing a population of the target exosomes, (S200) measuring quantitative signals from a plurality of biomarkers of the target exosomes present in the exosome liquid biopsy samples, and (S300) analyzing the exosome liquid biopsy samples from the normal and patient groups according to correlations between the plurality of biomarkers based on the measured quantitative signal values.

In S100, an exosome liquid biopsy sample is separated from an exosome bulk population sample from a normal group and an exosome liquid biopsy sample is separated from an exosome bulk population sample from a patient group. The exosome liquid biopsy samples are used as analytical samples. The exosome liquid biopsy samples from the normal and patient groups are separated from the respective exosome bulk population samples in the same manner.

Each of the exosome liquid biopsy samples includes a population of target exosomes captured from the corresponding exosome bulk population sample and associated with a specific disease. Specifically, each of the exosome liquid biopsy samples includes a subpopulation containing exosomes that belong to an exosome bulk population and have at least one specific biomarker as a target analyte in common, a sub-subpopulation containing exosomes that belong to the exosome subpopulation and have at least another specific biomarker as a target analyte in common, and a subset of exosomes that belong to the exosome sub-subpopulation and have at least another specific biomarker as a target analyte in common.

For clear understanding of the terms "exosome population", "exosome subpopulation", and "exosome sub-subpopulation" used herein, reference is made to CD63 and Cavl, which are melanoma cancer-related biomarkers. The exosome population is a bulk population containing exosomes released from cells in the body. The exosome subpopulation is a portion of the exosome population and contains target biomarkers as target analytes. A population of exosomes having CD63 markers, whose concentration was reported to increase in melanoma cancer samples, can be used as the exosome subpopulation. Since exosomes having Cavl markers exist in the exosome subpopulation having CD63 markers, a population of the exosomes having the Cavl markers is separated from the exosome subpopulation and can be used as the exosome sub-subpopulation. Conversely, a population of exosomes having Cavl markers may also be used as the exosome subpopulation and a population of exosomes belonging to the exosome subpopulation and having CD63 markers may also be used as the exosome sub-subpopulation.

In S200, quantitative signals from a plurality of biomarkers of the target exosomes present in the exosome liquid biopsy samples are measured. The biomarkers may include predetermined disease-associated biomarkers arranged on the surface of the exosomes and/or predetermined disease-associated biomarkers eluted from the exosomes. The concentration of the predetermined disease-associated biomarkers in a body fluid of a patient with a specific disease is higher than that in a body fluid of a healthy subject. Examples of the predetermined disease-associated biomarkers include proteins, mRNAs, tRNAs, miRNAs, long non-coding RNAs, DNAs, lipids, sugars, peptides, and other biochemical components that are involved in exosome biosynthesis in cells, exosome release from cells or exosome accommodation by recipient cells.

Quantitative signals from the biomarkers may be measured by any suitable measurement method known in the art, for example, enzyme-linked immunosorbent assay (ELISA). Other examples of methods for quantitative signal measurement include, but are not limited to, spectroscopy methods, electrochemical methods, electromagnetic methods, imaging methods, wave measurement methods, and other signal measurement methods based on sensor techniques.

In S300, biomarker profiling is performed on the exosome liquid biopsy samples. This analysis can be performed according to correlations between the plurality of biomarkers based on the measured quantitative signal values. Each exosome liquid biopsy sample can be analyzed according to a two-dimensional correlation between the quantitative signal from one biomarker and the quantitative signal from another biomarker. As an example, each exosome liquid biopsy sample can be analyzed based on the measured signal values in an XY coordinate system whose X-axis shows the quantitative signal from one biomarker and Y-axis shows the quantitative signal from another biomarker. A comparison of the distribution patterns between the exosome liquid biopsy samples from the normal and patient groups helps to discover biomarkers that can be used to diagnose a disease of the patient group. In addition, the stage of a disease such as cancer can be predicted by identifying an increased heterogeneity in the composition of biomarkers.

The analysis may also be performed by newly defining normalized parameters based on the quantitative signals. Here, each of the parameters can be defined as the ratio between the quantitative signal from one of the plurality of biomarkers as a reference signal and the quantitative signal from another biomarker. The number of the parameters is at least two and is set according to the number of the quantitative signals from the biomarkers other than the reference signal. For example, in the case where quantitative signals from four biomarkers are measured, parameters 1, 2, and 3 can be defined as the ratios of the signal from the second biomarker, the signal from the third biomarker, and the signal from the fourth biomarker to the signal from the first biomarker as a reference signal, respectively.

Here, the biomarkers used for the parameterization may be selected from the plurality of biomarkers whose quantitative signals have been measured. For example, the parameters may be set by coordinating the exosome liquid biopsy samples in a plurality of XY coordinate systems in which one or more of the quantitative signals of the X-axis and the Y-axis are different, as in the analysis according to a two-dimensional correlation between quantitative signals, calculating the slopes of the exosome liquid biopsy samples from the normal and patient groups, and selecting biomarkers constituting a XY coordinate system in which the slope difference is relatively large. That is, the parameters can be set using quantitative signals showing a significant difference by comparing the two-dimensional analytical results between the quantitative signals from the exosome liquid biopsy samples from the normal and patient groups.

When at least two parameters are set, the analysis may be performed according to a correlation between two different parameters. For instance, in a first XY coordinate system whose X-axis shows a first parameter and Y-axis shows a second parameter, the measured values are substituted for the first and second parameters to calculate the parameter values, the exosome liquid biopsy samples are coordinated according to the calculated parameter values, and the distribution patterns can be compared.

Here, a negative area in which the exosome liquid biopsy sample from the normal group is distributed can be set in the first XY coordinate system. In the case where the exosome liquid biopsy sample from the patient group is distributed in the negative area, an XY coordinate system between at least one of the first and second parameters and another parameter is configured for the exosome liquid biopsy samples in the negative area to reanalyze the distribution of the samples in the negative area. For example, positive accuracy *(i.e.* sensitivity) can be maximized by individually or sequentially reanalyzing the distribution in the negative area between the first and third parameters and the distribution of the negative area between the third and fourth parameters.

Likewise, a positive area in which the exosome liquid biopsy sample from the patient group is distributed can be set in the first XY coordinate system. In the case where the exosome liquid biopsy sample from the normal group is distributed in the positive area, the distribution of the exosome liquid biopsy samples in the positive area is reanalyzed in the same manner as described above. This reanalysis can maximize negative accuracy *(i.e.* specificity).

The biomarker profiling for the exosome sub-subpopulation is difficult to realize with existing immunoisolation techniques carried out under harsh conditions such as acidic pH when exosomes are recovered after immunoisolation of exosomal surface proteins. Accordingly, for sequential immunoisolation twice or more, it is difficult to use existing techniques such as chromatography, magnetic isolation, fluorescence-activated cell sorting (FACS), and microfluidic separation without modification. Exosome isolation techniques are still at the early stage of development. Indeed, exosomes are isolated currently by techniques such as simple, one-time immunoisolation based on exosomal surface proteins and fractionation based on the size, density, and solubility of exosome particles. There have been no reports on the diagnosis of specific diseases based on exosome sub-subpopulation separation by sequential immunoaffinity isolation and sub-subpopulation biomarker profiling.

According to the present invention, biomarker profiling on specific exosome subpopulations, sub-subpopulations, and subsets thereof in samples through sequential repeated isolation processes enables early diagnosis of specific diseases such as cancer diseases. This is because fundamental problems (e.g., damage to exosomes and low yield) caused by the use of harsh conditions such as acidic pH and/or a surfactant to recover specific exosomes captured by the immobilization members are solved. In conclusion, the present invention solves the problems of existing techniques, for example, difficulty in preserving the intact form of exosomes in the subpopulations upon recovery and low recovery rate, and thus enables the separation of target exosome sub-subpopulations associated with specific diseases through sequential repetitive separation and the diagnosis of a specific disease based on biomarker profiling.

Furthermore, when exosome subpopulations, exosome sub-subpopulations, and their subsets can be separated via specific exosomal surface proteins associated with specific diseases such as cancer diseases, as described above, nucleic acid biomarkers (e.g., mRNAs and miRNAs) for the specific diseases present at high concentrations in specific exosomes can be provided as liquid biopsy samples. Particularly, miRNAs present in exosomes, which are known to be protected from degradation by RNase and be stably detected in circulating plasma or serum, have emerged as ideal biomarkers for clinical diagnosis. Examples of miRNAs (miRs) found in plasma exosomes include ovarian cancer-associated miRNAs such as miR-21, -141, -200a, -200c, -203, -205, and -214, lung cancer-associated miRNAs, such as miR-7, -3p, -21, -20b, -223, -301, and let-7f, prostate cancer-associated miRNAs such as miR-141 and miR-375, breast cancer-associated miRNAs such as miR-21, cardiovascular disease-associated miRNAs such as miR-1 and miR-133a, and alcoholic liver disease-associated miRNAs such as miR-122 and miR-155. Examples of miRNAs found in urinary exosomes include miR-29c and CD2AP mRNA associated with renal fibrosis.

Most studies on exosome-derived miRNAs have focused on cancer diagnosis. These studies revealed that specific miRNAs are found at similar concentrations in ovarian cancer biopsy specimens or in serum exosomes isolated from the same ovarian cancer patient. However, such exosomal miRNAs are not found in normal groups, suggesting their use as potential diagnostic markers upon biopsy data collection. Prostate cancer can be early detected and diagnosed using prostate-specific antigen (PSA). However, PSA-based testing exhibits low diagnostic specificity and high false-positive rate, leading to overdiagnosis and overtreatment of prostate cancer. Thus, there is a need to develop new markers that exhibit higher diagnostic accuracy for prostate cancer. Some studies reveal that increased concentrations of miR-141 and miR-375 as miRNA (miR) markers are associated with tumor progression in prostate cancer. Some exosome-derived miRNAs such as miR-141 and miR-375 are expected to be valuable diagnostic markers for prostate cancer due to their RNase resistance in serum or plasma specimens.

miRNAs present in exosomes are potential diagnostic biomarkers for esophageal squamous cell cancer (ESCC). Several studies reveal that the concentration of exosome-derived miR-21 is elevated in serum from ESCC patients compared to in serum from patients with benign tumors without systemic inflammation and is clearly associated with tumor progression and aggressiveness. Moreover, exosome-derived miRNAs are potential diagnostic biomarkers for cardiovascular diseases and renal fibrosis.

In addition to miRNAs, exosomal mRNAs have potential utility as biomarkers in clinical diagnosis. Urinary exosomal samples from prostate cancer patients indeed contain mRNA of PCA3 and TMPRSS2, a product resulting from an ERG (v-ets avian erythroblastosis virus E26 oncogene homolog) fusion chromosomal rearrangement. Similarly, in urinary exosomes, diagnostic and prognostic markers of renal ischemia/reperfusion injury or antidiuretic hormone action, such as aquaporin-1 and -2, have been found.

Exosome subpopulation and sub-subpopulation samples prepared by immunoaffinity isolation techniques under mild conditions as described above may contain exosome-derived proteins and nucleic acids as diagnostic markers for specific diseases. Such potential proteins and nucleic acids are as follows.

A number of exosome-derived breast cancer markers such as CD24 and EpCAM have been clinically identified in recent years. In addition to breast cancer, exosomal proteins provide the useful source for biomarkers for a number of other cancers. Several studies revealed that survivin, a member of inhibitor of apoptosis (IAP), was detectable in plasma-derived exosomes from both normal and prostate cancer patients, but the relative amount of exosomal survivin is significantly higher in plasma of prostate cancer patients. Urinary exosomes, which are readily accessible by non-invasive means, have been utilized to develop biomarkers for prostate cancer and bladder cancer. Protein profiling of urinary exosomes from healthy and bladder cancer patients by label-free liquid chromatography-tandem mass spectrometry (LC-MS/MS) led to identification of urinary exosomal proteins as potential biomarkers for bladder cancer. PCA3 and TMPRSS2-ERG, two established prostate cancer markers, are also present in urinary exosomes from prostate cancer patients. The cell surface proteoglycan (protein-bound polysaccharide) was shown to be present in exosomes isolated by FACS from serum of pancreatic patients in both early and late stages, but not in benign pancreatic disease. Exosomes isolated by ultracentrifugation from ovarian cancer patients' plasma carried TGF-β1 and MAGE 3/6, but not in exosomes from patients with benign tumors.

Initial analyses of nucleic acids from isolated exosomes identified microRNAs (miRs) and mRNAs as the major components of exosomal cargo. Subsequent studies revealed that exosomes contain other species of RNAs such as transfer RNAs (tRNAs) and long noncoding RNAs (lnc RNAs). In addition to RNA species, fragments of both single stranded and double stranded DNAs were identified in exosomes. Exosomal miRNAs have been under the most attention among exosome-derived nucleic acids as cancer diagnosis biomarkers due to their stability against RNase-dependent degradation. Examples of exosomal nucleic acid biomarkers found in preclinical and clinical studies include: miR-21 and miR-1246 as exosomal nucleic acids in plasma applied to breast cancer diagnosis; miR-21, miR-141, miR-200a, miR-200c, miR-200b, miR-203, miR-205, and miR-214 as exosomal nucleic acids in serum applied to ovarian cancer diagnosis and screening; let-7a, miR-1229, miR-1246, miR-150, miR-21, miR-223, and miR-23a as exosomal nucleic acid in serum applied to colorectal cancer diagnosis; miR-141 and miR-375 as exosomal nucleic acids in plasma and serum applied to prostate cancer diagnosis; and miR-17-5p and miR-21 as exosomal nucleic acids in serum and urine applied to pancreatic cancer diagnosis.

In summary, cancer-derived exosomes contribute to cancer progression within the tumor microenvironment through enhancing intercellular transfer of cargo that contains proteins, lipids and nucleic acids. The cargo of exosomes reflects the altered state of original cancers, which qualifies exosomes as minimally invasive biomarkers for early detection, diagnosis and prognosis. In practice, exosome-based diagnostics provide higher sensitivity and specificity over conventional tissue biopsy or other biomarker-based liquid biopsy due to their stability in biofluids.

In addition, exosomal markers are readily available from most biofluids and recent technical advances in exosome isolation make exosome-based diagnostics cost and labor effective. The major obstacle to overcome in the field of exosome diagnostics is to develop optimal methods to isolate pure exosome population. MVEs themselves can be a useful diagnostic tool to detect cancers, but the comparison studies will only be possible with isolation techniques that separate MVEs into two major extracellular MVEs, pure exosomal vs. other microvesicle populations. Another challenge is to understand the mechanisms that regulate the heterogeneity of cancer exosomes, which will affect the contents of cancer-derived exosomal cargo, and therefore influence the reproducibility of diagnostic outcomes. Despite these concerns, future efforts that combine next generation sequencing of exosomal RNAs and DNAs, proteomic analysis of exosomal surface proteins, and immuno-affinity capturing techniques will enable to reach to the next level of exosome utilization for cancer diagnosis.

### Mode for Carrying out the Invention

The present invention will be more specifically explained with reference to the following examples, including evaluation examples. However, these examples are provided for illustrative purposes only and are not intended to limit the scope of the invention.

### I. Affinity isolation of exosomes based on switch-like binding reaction

### 1. Materials

The present inventors have produced monoclonal antibodies specifically recognizing the unique structure of altered calcium binding proteins (CBPs) bound with calcium ions. Mutated glucose-galactose binding protein (GGBPs) were selected as the CBPs. These proteins were produced from *Escherichia coli (E. coli)* and purified. Sodium chloride, trizma, casein (sodium salt form, extract from bovine milk), 3,3',5,5'-tetramethylbenzidine (TMB), sodium dodecyl sulfate (SDS), bovine serum albumin (BSA), and CNBr-activated Sepharose 4B gel (4% agarose) were purchased from Sigma (St. Louis, MO, USA). Calcium chloride dihydrate and potassium chloride were supplied by Daejung (Siheung, Korea). Sulfosuccinimidyl-6-[biotinamido]-6-hexanamido hexanoate (NHS-LC-LC-biotin), succinimidyl 4-(N-maleimidomethyl)cyclohexane-1-carboxylate (SMCC), N-succinimidyl 3-(2-pyridyldithio)-propionate (SPDP), dithiothreitol (DTT), maleimide, streptavidin (SA), and horseradish peroxidase (HRP) were purchased from Thermo Fisher Scientific (Rockford, IL, USA). Anti-CD63 antibody (Cat. No. 353014) was supplied by BioLegend (San Diego, CA, USA). Monoclonal antibodies specific for CD9 (Cat. No. MAB1880), CD81 (Cat. No. MAB4615) or Caveolin-1 (Cavl; Cat) No. MAB5736) were supplied by R&D Systems (Minneapolis, MN, USA). Lyophilized exosome standard reagents (human plasma- derived) and melanoma cancer cell cultures from healthy subjects were purchased from HansaBioMed (Tallinn, Estonia). Magnetic beads (Dynabeads M-280 Tosylactivated, 2.8 mm diameter) and streptavidin-poly-HRP20 were supplied by Invitrogen (Carlsbad, CA, USA) and Fitzgerald (Acton, MA, USA), respectively. Several other exosome isolation kits, including ExoQuick^{™} kit (System Biosciences; Mountain View, CA, USA), MagCapture^{™} kit (Wako; Osaka, Japan), and exoEasy Maxi^{™} kit (Qiagen; Hilden, Germany), were purchased from different sources. Other reagents used were of analytical grade.

### 2. Example 1: Preparation of components for exosome isolation and characterization

### 2.1 Conjugation between SA and CBP

CBP (400 µg) was dissolved in a 10 mM phosphate buffer (pH 7.4; PB) and mixed with 20-fold molar excess of SMCC. The mixture was allowed to react at room temperature for 1 h to activate CBP. SA (2 mg) dissolved in PB was reacted with 5-fold molar excess of SPDP for 1 h and chemically reduced with 10 mM DTT solution containing 5 mM ethylenediaminetetraacetic acid (EDTA) at 37 °C for 1 h to expose sulfhydryl groups. The activated components were separated on a Sephadex G-15 gel filtration column (10 ml volume) to remove excess reagents. The two activated proteins *(i.e.* maleimide-CBP and thiolated-SA) were mixed in a 1:5 molar ratio. The mixture was allowed to react at room temperature for 4 h. Thereafter, the final solution containing the CBP-SA polymer was stored at 4 °C in a tightly-sealed container before use.

### 2.2 Biotinylation of anti-CD63 antibody

The succinimidyl-ester moiety of NHS-LC-LC-biotin was reacted with the amine group on the antibody molecule to biotinylate the antibody. Briefly, anti-CD63 antibody dissolved in PB solution containing 140 mM NaCl (PBS) was mixed with NHS-LC-LC-biotin dissolved in 20-fold molar excess of dimethyl sulfoxide (DMSO). The mixture was allowed to react at room temperature for 2 h. Unreacted reagents in the reaction mixture were removed by size-exclusion chromatography using a Sephadex G-15 column equilibrated with PBS. The purified conjugate was dialyzed twice against PBS, concentrated to 1 mg/ml with Vivaspin 500, and stored at 4 °C before use.

### 2.3 Labeling of anti-CD63 antibody with HRP

Anti-CD63 antibody was labeled with HRP according to the general procedure. Briefly, first, the antibody was reduced with 10 mM DTT at 37 °C for 1 h and excess reagent was removed with a gel filtration column. HRP was activated with 30-fold molar excess of SMCC and unreacted reagent was removed by size-exclusion chromatography as before. After the reduced antibody was mixed with 10-fold molar excess of the activated HRP, conjugation was performed at 4 °C for ≥ 24 h. The resulting conjugate was mixed with an equal volume of glycerol and stored at -20 °C.

### 3. Example 2: Immunoaffinity chromatography

### 3.1 Preparation of immunoaffinity gel

For immunoaffinity chromatography, the CBP conformation-specific monoclonal antibody was immobilized on the surface of the CNBr-activated Sepharose 4B gel. Lysine residues on the antibody molecule were reacted with functional groups on the gel under alkaline conditions to chemically bind the antibody (2 mg per ml of the hydrated gel) to the agarose gel according to the manufacturer's instructions. After antibody binding, the remaining reactive groups on the gel were hydrolyzed with Tris-HCl buffer.

The resulting immunoaffinity gel was packed in a plastic column to fabricate an immunoaffinity chromatography column, which was then washed under alternating acidic and alkaline conditions to remove the antibody non-specifically adsorbed to the solid (gel) surface.

### 3.2 Fabrication of immunoaffinity chromatography column and isolation of CD63 positive (CD63+) exosomes through the column

The immunoaffinity gel was used to fabricate an immunoaffinity chromatography column. An immunoaffinity chromatography column specific to CD63-positive (CD63+) exosomes was fabricated by the following procedure. First, a plastic column was packed with the immunoaffinity gel. The immunoaffinity gel was immobilized with the CBP-SA conjugate (5 µg/ml) by reacting with the CBP conformation-specific monoclonal antibody using a transport solution containing Ca²⁺ (10 mM) ("Ca²⁺ switch-on" condition). Thereafter, the biotinylated anti-CD63 antibody (3 µg/ml) was immobilized on the CBP-SA conjugate layer via biotin-SA binding.

The fabricated immunoaffinity column (9 mm × 3.2 mm; 1.5 ml bed volume) was used for exosome isolation. Specifically, a standard exosome sample from a healthy subject (25 µg/ml; 1 ml) was loaded onto the CD63 immunoaffinity column. A Ca²⁺-containing transport solution was injected to wash off unbound components. Thereafter, a Ca²⁺-free solution ("Ca²⁺ switch-off condition) was injected to recover fractions of the CBP-antibody-exosome complex (containing CD63+ exosomes) bound to the immunoaffinity gel. Here, the immunoaffinity column was regenerated and reused for analysis of other samples.

More specifically, the gel was initially equilibrated with 20 mM Tris-HCl containing 140 mM NaCl, 10 mM Ca²⁺, and 2% BSA (pH 7.4; binding solution) before addition of an exosome sample to the immunoaffinity column. Each of the CBP-SA (5 µg/ml, 1 ml) and the biotinylated anti-CD63 antibody (3 µg/ml, 1 ml) prepared in Example 1 and an exosome standard sample (1 ml; normal exosome standard reagent or melanoma cancer cell culture) was diluted with a binding solution. The HRP enzyme (0.1 µg/ml) was added to each dilution for marking the addition position of each reagent. The resulting solutions were sequentially added to the gel column such that they were eluted at predetermined elution volumes. The eluate from the column was transferred to a fraction collector (fraction volume: 1 ml) using a peristaltic pump at an elution rate of 120 µl/min. Unreacted proteins were removed from the gel column by washing with a BSA-free binding solution. Finally, a 20 mM Tris-HCl buffer solution (pH 7.4) containing 500 mM NaCl was fed into the column to elute and recover exosomes bound to the column. Thereafter, a 100 mM sodium acetate buffer (pH 4.5) containing 500 mM NaCl, 5 % (v/v) Tween-20, and 0.02 % (w/v) thimerosal was fed into the gel column for regeneration, and the column was thoroughly washed until no protein elution was observed.

### 4. Example 3. Immunomagnetic isolation

### 4.1 Immunomagnetic enrichment of CD63+ exosomes

Generally, magnetic enrichment is a method for separating and concentrating a target substance through an antigen-antibody reaction using antibody-bound immunomagnetic beads under a magnetic field. The isolation of CD63+ exosomes based on this method is basically the same as the immunoaffinity chromatography performed in Example 2. The magnetic beads were bound with the monoclonal antibody recognizing a conformational change of CBP by Ca²⁺ binding, followed by sequential reaction with the CBP-SA conjugate and the biotinylated anti-CD63 antibody under the "Ca²⁺ switch-on" condition. The resulting immunomagnetic beads were allowed to react with an exosome sample (normal exosome standard sample (25 µg/ml; 1 ml)). The magnetic beads were captured with a magnet, the supernatant was removed, and the beads were washed. Then, the buffer solution was added under the "Ca²⁺ switch-off' condition to recover and concentrate CD63+ exosomes. The volume of the buffer solution used was one-tenth of its initial volume.

More specifically, for magnetic enrichment, the amine groups on the CD63-specific antibody were chemically bound to and immobilized onto the activated tosyl moieties on the solid surfaces of the magnetic beads. After washing 3 times with Tris-HCl buffer, the remaining surfaces were blocked with casein-PBS. After magnetic separation of the beads (total 1 mg) from the solution, the CBP-SA conjugate (5 µg/ml; 1 ml) diluted with a binding solution was added and incubated on a shaker for 1 h. After washing the beads with a BSA-free binding solution, the biotinylated anti-CD63 antibody (3 µg/ml; 1 ml) was incubated under the same conditions. After washing again, an exosome sample (25 µg/ml; 1 ml) diluted with a binding solution was added and allowed to react for 3 h. Finally, CD63+ exosomes were recovered and the beads were regenerated according to the same protocol as in immunoaffinity chromatography.

### 5. Evaluation Example 1: Analysis of the eluent fractions recovered in Example 2

Fig. 15 shows the results of immunoaffinity chromatography for CD63-positive (CD63+) exosomes in the sample based on switch-like binding.

First, a chromogenic enzyme assay for horseradish peroxidase (HRP) was conducted to determine the elution positions of the reagents added in Example 2 on the chromatogram. Analysis of the eluent fractions collected from the column during elution in Example 2 confirmed the addition position of each component and the distribution of CD63+ exosomes in each fraction. When each of the components *(i.e.* the CBP-SA conjugate, the biotinylated anti-CD63 antibody, and the exosome sample) was injected into the pretreated gel column, each solution was allowed to contain a total of 0.1 µg of HRP such that the addition position after elution was determined. To this end, portions of each fraction were sampled and plated in microwells and an HRP substrate solution was added thereto. From the results, a chromatogram for information on the injection locations of the components was obtained. Peaks (a), (b), and (c) are shown by dotted lines in Fig. 15. More specifically, for the enzyme assay, a predetermined amount (5 µl) of each fraction was plated in a microtiter plate and then an enzyme substrate solution (200 µl) was added to generate a signal from the enzyme. The enzyme substrate solution was prepared by mixing a 3% (v/v) aqueous hydrogen peroxide solution (10 µl), 10 mg/ml TMB (100 µl) dissolved in dimethyl sulfoxide (DMSO), and a 0.05 M acetate buffer (pH 5.1; 10 ml). The reaction was stopped with the addition of a 2 M sulfuric acid solution (50 µl) before saturation of the enzyme signal. The optical density was measured with a microplate reader (Synergy H4, BioTek Inc.; Winooski, VT, U.S.A.) at a maximum absorbance of 450 nm.

Furthermore, enzyme-linked immunosorbent assay (ELISA) was conducted on exosomes in each fraction to determine the elution position of the exosomes isolated and recovered via immunoaffinity chromatography. To this end, portions of each fraction were sampled and plated in microwells immobilized with the anti-CD63 antibody, followed by incubation. Thereafter, the SA-poly HRP 20 conjugate was allowed to react with the remaining biotin on the biotinylated antibody molecules predicted to react with exosomes in the column. More specifically, 5 µg/ml antibody (100 µl) was added to each well and incubated at 37 °C for 1 h for antibody immobilization. After washing, the remaining surfaces were blocked with Tris-HCl buffer containing 0.5% casein (casein-Tris). Then, an aliquot (100 µl) of each fraction was added to each well and incubated in a container maintained at 37 °C and a humidity of 100% for 12 h. The wells were washed and the biotinylated anti-CD63 antibody (1 µg/ml; 100 µl) prepared in Example 1 was added thereto, followed by incubation at 37 °C for 1 h. After washing again, SA-poly HRP20 (66 ng/ml; 100 µl per well) diluted with casein-Tris was added and incubated at 37 °C for 1 h. Finally, a substrate solution for HRP (200 µl) was added and the reaction was stopped with the addition of a 2 M sulfuric acid solution (50 µl) before color development was saturated. The optical density was measured as above. The exosome-specific chromatography results obtained by adding the HRP substrate solution are shown by solid lines in Fig. 15. Significant signals under the "Ca²⁺ switch-off' condition are indicated by a peak (d) in Fig. 15. Since only the anti-CD63 antibody sandwich complexes formed around the CD63+ exosomes were detected by the immunoassay (see the inset in Fig. 15), the peak (d) eluted from the column was determined to contain CD63+ exosomes separated and recovered by immunoisolation. In addition, the generation of weak signals even at the injection location of the exosome sample (peak (c) in Fig. 15) was predicted as a result of elution of some exosome complexes or non-specific reaction of excess exosomes having no affinity for the column. However, the peak (c) was excluded from further analysis because its size was less than 10% of that of the peak (d).

### 6. Evaluation Example 2: Evaluation of reproducibility of the immunoaffinity chromatography of Example 2

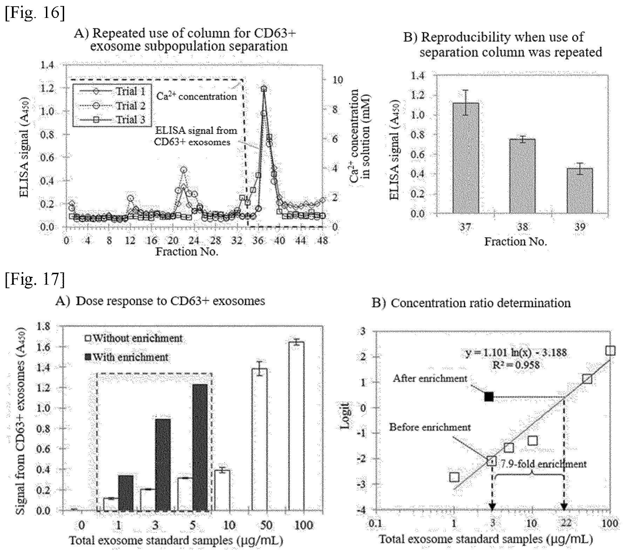

Fig. 16 shows the reproducibility of exosome isolation when the immunoaffinity chromatography of Fig. 15 was repeated.

To confirm the reproducibility of exosome isolation using the immunoaffinity column of Example 2, the same immunoisolation procedure was repeated three times. As already mentioned above, eluent fractions obtained from each experiment were subjected to immunoassay on CD63+ exosomes to obtain their chromatograms (see Fig. 16, A). After exchange with a Ca²⁺-free transport solution for elution (after the 35^{th} fraction), CD63+ exosomes were isolated and formed single independent peaks despite repeated experiments. Moreover, the fraction numbers forming the CD63+ exosome peaks and the intensities of specific signals from exosomes were found to be very similar. Particularly, the immunoassay signals from CD63+ exosomes present in the fraction Nos. 37, 38, and 39 in the recovered exosome peaks on the chromatogram showed an average coefficient of variation (CV) of ≤ 9.4%, indicating high reproducibility (Fig. 16, B).

More specifically, first, an immunoaffinity chromatogram was obtained as above and exosomes present in each eluent fraction were recovered and analyzed according to the same isolation protocol. This procedure was repeated three times. The results of immunoassay for CD63+ exosomes present in the main fractions constituting each peak for isolated exosomes were obtained. The coefficient of variation (CV) between the measured signals was used as an index for the evaluation of reproducibility for isolation tests. The gel column for isolation was filled with Tris-HCl buffer and stored at 4 °C when not in use.

### 7. Evaluation Example 3: Evaluation of enrichment performance of Example 3

Fig. 17 shows the isolation and enrichment of CD63+ exosomes in the sample by immunomagnetic isolation based on switch-like binding.

To test the enrichment performance of Example 3, magnetic isolation was repeatedly applied to other standard exosome standard samples (1, 3 or 5 µg/ml), followed by sandwich enzyme-linked immunosorbent assay on CD63+ exosomes, as described above. The results are shown as black bars in Fig. 17, A. The results of immunoassay for CD63+ exosomes in the original standard samples before magnetic enrichment are shown in white bars in Fig. 17, A. Comparing the results of the samples before and after magnetic isolation, the intensities of signals generated by magnetic enrichment increased by an average of about 3.7 times. The dose responses of the immunoassay for the original standard samples were linearized by log-logit transformation to determine the enrichment ratio of the isolated exosomes and the concentrations of the enriched CD63+ exosomes were determined therefrom (Fig. 17, B). Particularly, the 3 µg/ml exosome sample was enriched to 22 µg/ml based on CD63+ exosomes, which corresponds to an enrichment efficiency of approximately 7.9 times.

The concentrations of the exosome samples before and after isolation were determined by comparison with the results obtained with the standard reagent for immunoassay. First, the normal exosome standard reagent was diluted with casein-Tris to prepare exosome standard samples (1-100 µg/ ml). Anti-CD63 antibody (5 µg/ml; 100 µl) was plated in microwells for immunoassay, incubated at 37 °C for 1 h, and immobilized on the inner surfaces of the wells with a capture binder. After washing, the remaining surfaces were blocked with casein-Tris under the same conditions as above. The exosome samples (each 100 µl) were plated in different wells and incubated at 37 °C for 12 h. The subsequent procedure was performed according to the ELISA protocol described above.

### 8. Evaluation Example 4: Evaluation of reproducibility of the immunomagnetic isolation of Example 3

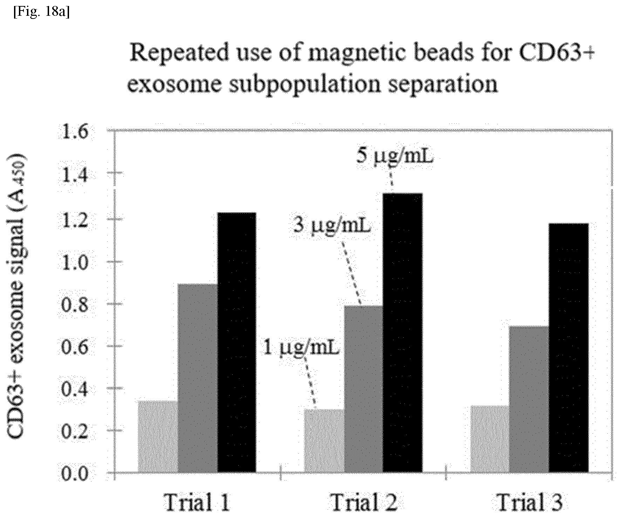

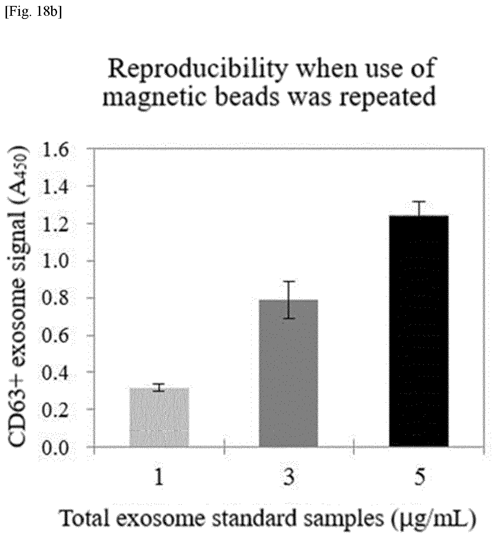

Fig. 18 shows the reproducibility of exosome isolation when the immunomagnetic isolation of Fig. 17 was repeated.

The magnetic enrichment technique based on the switch recognition material in Example 3 enables capture of target exosomes under the "Ca²⁺ switch-on" condition, sequential recovery of exosomes under the "Ca²⁺ switch-off' condition, regeneration of pretreated magnetic beads, and reuse of pretreated beads for isolation and enrichment of new samples. To evaluate the reproducibility of enrichment performance for actual reuse of pretreated beads, different concentrations of exosome standard samples (1, 3, and 5 µg/ml; collected from normal human plasma) were magnetically isolated and enriched and the same procedure was repeated twice using the reused pretreated beads. The concentrations of the isolated exosome samples were analyzed by sandwich enzyme-linked immunosorbent assay for CD63+ exosomes. The results are shown in Fig. 18, A. Referring to Fig. 18, A, the concentration response patterns for CD63+ exosomes were very similar when the samples were isolated and enriched using the pretreated beads. In practice, the enrichment of CD63+ exosomes in the isolated samples depending on the exosome concentration was found to be very reproducible with an average CV of 8.3% based on the signal values from immunoassay (see Fig. 18, B).

Here, the standard samples (1, 3, and 5 µg/ml) were prepared by diluting an exosome standard reagent from a healthy subject with a binding solution. The magnetic beads were immersed in casein-Tris and stored at 4 °C when not in use.

### 9. Evaluation Example 5: Characterization of CD63+ exosomes isolated in Examples 2 and 3

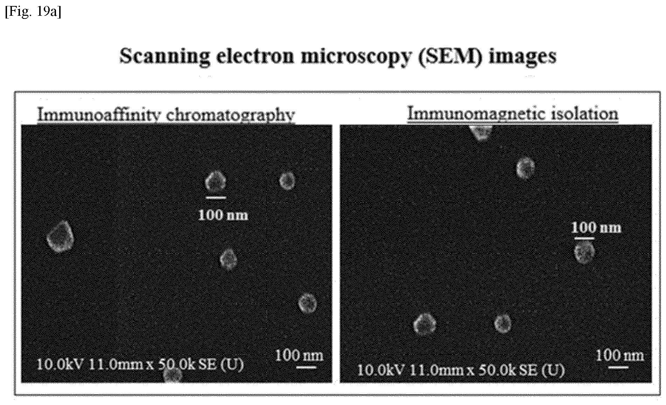

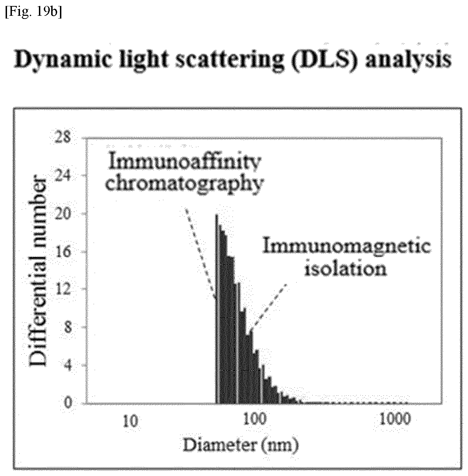

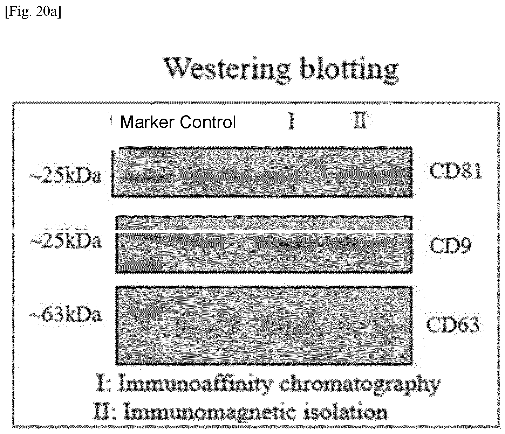

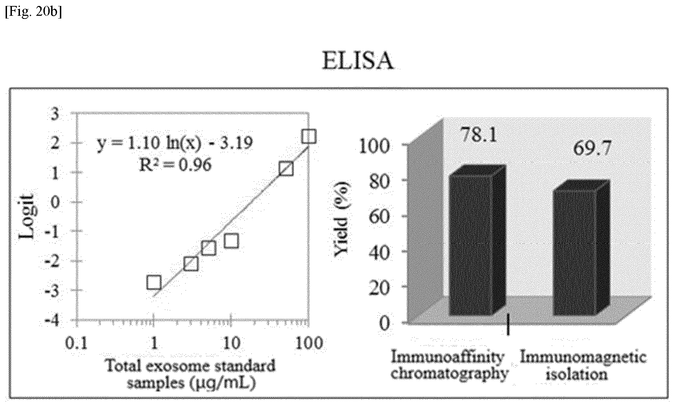

Fig. 19 shows the results of physical characterization for CD63+ exosomes isolated based on switch-like binding reaction. Fig. 20 shows the results of immunochemical characterization for exosome surface markers of CD63+ exosome samples separated based on switch-like binding reaction.

Two characteristics (including morphology of microvesicles and distribution of different types of surface proteins) of exosomes were actually determined through morphological characterization of the CD63+ exosome subpopulations separated in Examples 2 and 3 and immunoassay for surface proteins. For morphological characterization, the morphology of exosomes isolated by the inventive method was measured. To this end, images were observed using a scanning electron microscope (SEM) and the size distribution was measured by dynamic light scattering (DLS). The distribution of exosomal proteins was determined by Western blotting. The recovery rate after isolation was measured by enzyme-linked immunosorbent assay (ELISA).

### 9.1 Scanning electron microscopy (SEM) imaging

The morphologies of the CD63+ exosome samples isolated in Examples 2 and 3 were observed by SEM imaging. To obtain high magnification images, specimens were produced from the exosome samples separated by immunoaffinity chromatography or immunomagnetic isolation according to the standard protocol. To measure the solid-state microstructure and morphology, each original sample was transferred to a microscope glass slide and covered with a coverslip, and the object was immersed in liquid nitrogen and fixed by quick freezing. After removal of the coverslip, the frozen specimen was fixed by the addition of a 2.5% glutaraldehyde solution, washed with Tris-HCl buffer and deionized water (each once), and dehydrated with acetone. Then, acetone was removed using critical-point drying with liquid carbon dioxide. The samples were mounted on aluminum stubs using a tempfix mounting adhesive, and their surfaces were brought into contact with colloidal silver. After platinum was sputter-coated to a thickness of 3-5 nm, the samples were observed with a Hitachi S-4700 SEM (Hitachi; Tokyo, Japan). In the image of each sample, spherical particles with a diameter of -100 nm were observed (see Fig. 19, A). In practice, exosomes are membrane microvesicles released from various types of cells and are known to have a diameter of approximately 30-200 nm.

### 9.2 Dynamic light scattering (DLS) analysis

To more accurately measure the size distribution of each separated CD63+ sample, DLS analysis was conducted on exosomes using a particle size analyzer (ELSZ-1000, Otsuka Electronics; Osaka, Japan) at room temperature. The exosome sample (0.5 ml) was transferred to a disposable cuvette (BI-SCP, Brookhaven Instruments Corporation; NY, USA) and analyzed according to the manufacturer's guidelines. After the size of exosomes was determined according to the Stokes-Einstein equation, the size distribution of extracellular vesicles was characterized. As a result, the average particle diameter in each sample was found to be at the level of -186.2 nm or -193.8 nm (see Fig. 19, B). From the measured particle size and particle size distribution, it is believed that the particles present in the sample separated by immunoaffinity chromatography and immunomagnetic isolation are exosomes.

### 9.3 Western blotting

To determine the distribution of different types of surface proteins, which is one of the general characteristics of exosomes, Western blotting was conducted on tetraspanin protein markers in the CD63+ exosome samples separated in Examples 2 and 3. After SDS-PAGE analysis was conducted on the exosome samples under optimized conditions, the isolated proteins were transferred to a nitrocellulose membrane and antibody-HRP conjugates specific for CD9, CD63, and CD81, which are usually abundantly distributed on the exosome surface, were allowed to react with the respective target proteins. More specifically, first, each exosome sample was treated with a 50 mM Tris buffer (pH 8.0) containing 150 mM NaCl, 1% Triton X-100, 0.5% sodium deoxycholate, and 0.1% sodium dodecyl sulfate (SDS). The denatured proteins were isolated using 8% SDS-polyacrylamide gel electrophoresis (SDS-PAGE) and transferred onto a nitrocellulose membrane by electrophoresis. After the remaining surfaces were blocked with casein-PBS, blotting was conducted using anti-CD63, CD9, and CD81 antibodies, which had previously been chemically conjugated with HRP Finally, a HRP substrate solution containing 3,3'-diaminobenzidine (DAB) as a chromogenic reagent was added to generate colorimetric signals on the membrane. The substrate solution was prepared by adding 0.05% DAB (10 µl) and 3% H₂O₂ (1 µl) to a 50 mM sodium acetate buffer (pH 5.0; 1 ml).

The stained colorimetric signals demonstrated that all tetraspanin protein markers of exosomes were expressed in the CD63+ exosome samples separated in Examples 2 and 3 (see Fig. 20, A). For reference, the band signals from CD63 were not clear compared to the colorimetric signals of the bands for CD9 and CD81. This was affected by the relatively wide distribution of the bands (30-60 kDa) because the CD63 markers are glycosylated proteins, but the more fundamental reason was that the band signal for the expression of CD63 markers in the exosome standard sample (Fig. 20, A; Control) used in this evaluation experiment was low (see Fig. 20, A). Taken the above results together, the samples separated by immunoaffinity chromatography and immunomagnetic isolation exhibit all general characteristics of exosomes, indicating that CD63+ exosome subpopulations were successfully separated by the inventive method.

### 9.4 Recovery rate of CD63+ exosomes after isolation

To determine the recovery rates of exosome isolation systems, CD63+ exosomes in the samples were quantitatively analyzed before and after isolation and the concentrations of the CD63+ exosome subpopulations were indirectly measured using a standard curve obtained through immunoassay on CD63 markers for the exosome standard samples. The standard curve was obtained by enzyme-linked immunosorbent assay (ELISA) using an antibody specific to CD63, an exosome surface marker, as a capture and detection binder during immunoassay (Fig. 20, B; left). Immunoassay was conducted on the samples under the same conditions before and after isolation. The resulting signals were substituted into the standard curve to calculate the concentrations of exosomes. After calibration of the volume dilution factor, the total amounts of exosomes in the exosome samples before and after immunoaffinity chromatography and immunomagnetic isolation were calculated to determine the recovery yields of the isolation processes (Fig. 20, B; right). The yields of immunoaffinity chromatography and immunomagnetic isolation were found to be ~78.3% and ~68.5%, respectively, which were very high compared to the yields of conventional isolation processes (*e.g., <* 50%) for recovering isolation products under severe conditions such as acidic pH. This is because the affinity of the recognition material for CBP was lowered to the background level when calcium ions were removed from the solution in the isolation process using the switch recognition material, resulting in relatively high recovery yield of the isolated exosomes and minimal structural deformation of exosomes.

### 10. Evaluation Example 6: Evaluation of analysis performance when CD63+ exosome subpopulations were used as diagnostic samples

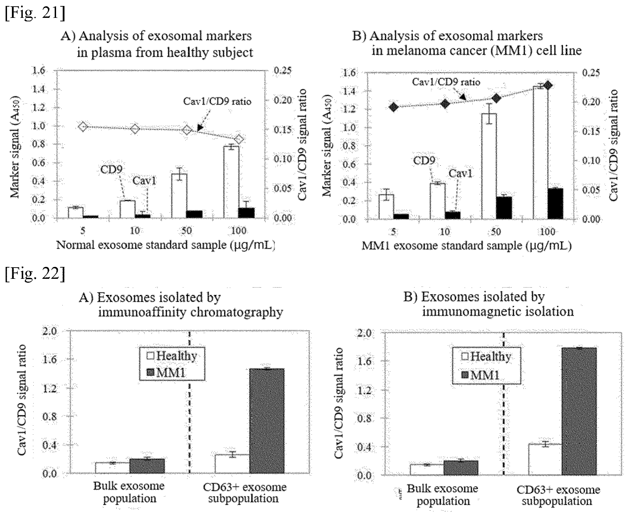

Fig. 21 shows the corrected concentrations of Cavl markers based on the concentration of CD9 to compensate for variations in concentration between exosome standard samples. Fig. 22 shows the potential effects of the use of CD63+ exosomes isolated by the inventive affinity isolation system and method based on switch-like binding reaction as analytical samples on melanoma cancer diagnosis.

### 10.1 Concentration normalization of target markers

When the separated CD63+ exosome subpopulations were used as diagnostic samples, high-sensitivity diagnosis is predicted due to the enrichment effect of Cav1+ exosomes as melanoma cancer-associated markers. However, when the expression level of Cavl protein is simply measured, there is a limitation in accurately determining the isolation effect due to the different concentrations of exosomes in the samples. Hence, a method for normalizing the marker through calibration based on CD9 normally present on the surface of exosomes was introduced to minimize uncertainty during performance comparison. To this end, Cavl protein-specific antibodies and CD9 protein-specific antibodies were immobilized onto different wells of a microtiter plate, the same sample was plated in the wells, followed by incubation. ELISA was performed using CD63 antibodies as detection antibodies. As a result, signals proportional to the concentrations of the cancer markers and the normal markers were obtained (see the bars in Fig. 21) and the ratios of the signals (that is, Cav1/CD9 signal ratios) were calculated (see the line in Fig. 21). When a plasma sample from a healthy subject was diluted and analyzed (Fig. 21, A), the Cav1/CD9 signal ratios were, on average, 0.147, which was maintained almost constant at different dilution factors. For melanoma cancer cell samples (Fig. 21, B), relatively high signals were measured for the markers and the Cav1/CD9 ratios were, on average, 0.206, indicating that the Cavl expression levels were ~40% higher on average than those of the standard samples. As such, changes in total exosome concentration could be normalized by calibrating Cav1+ exosomes in the CD63+ exosome subpopulations with CD9+ exosomes as normal markers.

To elaborate on the evaluation experiment procedure, the exosome standard samples (5-100 (µg/ml) were prepared by diluting exosomes obtained from normal plasma or a melanoma cancer cell line with casein-Tris. The concentrations of markers in the samples were measured as follows. First, capture antibodies (5 µg/ml; 100 µl) specific for the markers were plated in microwells and incubated at 37 °C for 1 h for immobilization. The subsequent procedure was performed in the same manner as in the ELISA procedure for CD63 described above. Signals generated after analysis for CD9 and Cavl were measured and the Cav1/CD9 signal ratios were calculated for the samples.

### 10.2 Potential of separated exosome subpopulations as diagnostic samples

The potential effects of the CD63+ exosome subpopulations as diagnostic samples separated based on switch recognition materials on the diagnosis of melanoma cancer were tested using the method for measuring the Cav1/CD9 signal ratio by immunoassay. Healthy exosome and melanoma cancer exosome standards (each 25 µg/ml) were separated to obtain CD63+ exosome subpopulations and the expression levels of Cavl protein were compared with those before separation. The Cav1/CD9 signal ratio of the bulk cancer exosome sample before separation was increased by ~40% compared to that of the bulk healthy exosome sample before separation (Fig. 22, A; left). The signal ratio of the cancer CD63+ exosome subpopulation sample separated by immunochromatography was increased by ~4-fold *(i.e.* 400%) compared to that of the healthy CD63+ exosome subpopulation sample separated by immunochromatography (Fig. 22, A; right). That is, the use of the separated CD63+ exosome subpopulation sample resulted in a ~ 10-fold increase in potential sensitivity of melanoma cancer cell screening. The increased potential sensitivity to melanoma cancer cell screening was also found for the use of the subpopulation sample separated by immunomagnetic isolation (Fig. 22, B). This effect is predicted to be due to the use of the CD63+ exosome subpopulation as a screening sample that leads to not only an increase in the concentration of Cav1+ exosomes but also a decrease in the concentration of CD9+ exosomes. It has indeed been reported that the concentrations of CD9+ exosomes are reduced in cancer samples.

### 11. Evaluation Example 7: Performance comparison with conventional exosome isolation systems

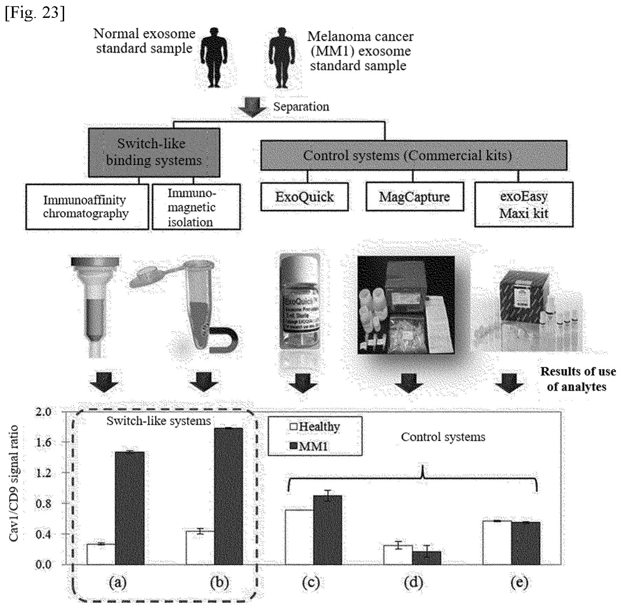

Fig. 23 compares the abilities of the affinity isolation systems based on switch-like binding reaction to distinguish melanoma cancer samples from normal samples based on a Cav1/CD9 concentration ratio determined by immunoassay of the separated samples with those of currently commercially available exosome isolation systems.

The effects of the CD63+ exosome subpopulation samples obtained using the switch recognition material-based isolation systems in Examples 2 and 3 on melanoma cancer cell screening were objectively evaluated by comparison with those of samples obtained using currently commercially available exosome isolation kits. The commercially available exosome isolation kits were sold under the trade names ExoQuick^{™}, MagCapture^{™}, and exoEasy Maxi^{™}. Twenty five µg/ml normal exosome and melanoma cancer exosome standards (each 1 ml) were isolated according to the established protocol of each system. The expression levels of Cavl and CD9 proteins in the isolated exosome samples were measured as signals by ELISA, as described above. The Cav1/CD9 signal ratios as calibration parameters were calculated and compared. The results are shown in Fig. 23. Referring to Fig. 23, the Cav1/CD9 signal ratios of the cancer CD63+ exosome subpopulation samples obtained by the inventive immunochromatography and immunomagnetic isolation systems were increased by 5.5-fold and 4.1-fold in a cancer sample, respectively, compared to those of the normal samples, as mentioned above. In contrast, there were no substantial differences in Cav1/CD9 signal ratio between the samples obtained using the commercially available isolation kits and the samples before separation, regardless of the kits used. Rather, the Cav1/CD9 signal ratios of the cancer samples were decreased compared to those of the normal samples.

These results are thought to be because the isolation principles of the commercial exosome isolation kits are different from the principles of the inventive isolation systems. The commercial kits are based on a precipitation method for exosome isolation using a polymer such as polyethylene glycol (ExoQuick^{™}, SBI), an affinity method for exosome isolation using a protein reacting with phosphatidylserine (PS) on the exosome surface in the presence of metal ions (MagCaprue^{™}, Wako), and a method for exosome isolation using a spin column to shorten the time required for isolation (exoEasy Maxi^{™}, Qiagen).

Most of the commercial kits are only used to separate or concentrate bulk exosome populations and are limited in significantly improving diagnostic sensitivity, making it difficult to apply to cancer cell screening. In addition, the conventional immunoisolation targeting specific exosome surface markers can be applied to increase diagnostic sensitivity but is not competitive in terms of exosome recovery yield and maintenance of intact form without introducing an innovative method using a switch recognition material as in the present invention.

Hereinafter, processes for exosome isolation using the commercial kit used in this evaluation experiment will be described briefly.

### Exosome isolation using ExoQuick kit:

Exosome standard samples were isolated according to the manufacturer's guidelines. Briefly, commercial exosome samples (each 25 µg/ml; 1 ml) were separately transferred into microwells, and predetermined amounts of ExoQuick exosome precipitation solution were added thereto. After mixing, the mixtures were refrigerated at 4 °C. The mixtures were centrifuged and the supernatant was discarded. The exosomes precipitated in the form of granules were collected, redissolved in a buffer (500 µl), and subjected to immunoassay to determine the Cav1/CD9 ratio as described already.

### Exosome isolation using MagCapture kit:

The same exosome standard sample was isolated according to the manufacturer's protocol. Briefly, magnetic beads (60 µl) coated with phosphatidylserine (PS)-bound protein, a specific capture factor for exosomes, were added to a sample (1 ml) containing metal ions as binding promoters. The mixture was allowed to react with stirring for 3 h. Thereafter, the beads were washed three times with a washing solution (2 mM CaCl₂ containing 150 mM NaCl, 0.05% Tween20, and 20 mM Tris-HCl (pH 7.4; 1 ml)) and eluted with an eluent (20 mM Tris-HCl containing 150 mM NaCl and 2 mM EDTA (pH 7.4)). Immunoassay was conducted to determine the Cav1/CD9 ratio for the recovered exosome sample.

### Exosome isolation using exoEasy Maxi kit:

An exosome sample was isolated using another commercial kit. Briefly, XBP buffer (1 ml) provided with the kit was added to an equal volume of the sample. After mixing gently, the mixture was left standing at room temperature and preheated to room temperature. The mixture was transferred into an exoEasy spin column, followed by centrifugation for 1 min. After XWP buffer solution (10 ml) was loaded onto the column, centrifugation was performed for additional 5 min to remove the loaded buffer solution. The spin column was transferred to a new recovery tube and XE buffer (500 µl) was then loaded onto the column, followed by incubation for 1 min. After centrifugation for additional 5 min, exosomes were eluted and recovered in a tube. Finally, the eluate was again loaded onto a spin column, incubated for 1 min, and centrifuged for 5 min. The eluate was recovered and subjected to immunoassay to determine the Cav1/CD9 ratio as mentioned above.

### II. Evaluation of usefulness of CD63+ subpopulation separation for cancer diagnosis

### 1. Materials

Sensors and sensor systems were purchased from ForteBio (San Francisco, USA) and used in the following examples. Other materials were the same as those used in the section "I. Affinity isolation of exosomes based on switch-like binding reaction".

### 2. Biomarker analysis using label-free sensors

To support the usefulness of CD63+ subpopulation separation for cancer diagnosis, a bulk exosome standard sample and an exosome subpopulation sample obtained after separation were simultaneously measured for CD9 and Cavl markers, respectively, using label-free sensors (Octet sensor; ForteBio). The results are shown in Figs. 24 to 27.

Figs. 24 to 27 show the experimental results obtained when CD9 and Cavl markers in samples before and after immunoaffinity isolation of the exosome standard samples were simultaneously measured using label-free sensors in order to verify the effect of exosome subpopulation separation. Specifically, Fig. 24 shows the concentrations of CD9 in a normal exosome sample before and after separation, Fig. 25 shows the concentrations of Cavl in a normal exosome sample before and after separation, Fig. 26 shows the concentrations of CD9 in a cancer cell exosome sample before and after separation, and Fig. 27 shows the concentrations of Cavl in a cancer cell exosome sample before and after separation.

First, CD9-containing exosomes in a normal sample were measured before and after isolation. To this end, sensors immobilized with a specific capture antibody to CD9 were immersed in the exosome sample and signals were measured in real time for 1,200 sec (see Fig. 24). The signals associated with CD9 from the original sample before isolation were measured. As a result, the average binding signal increased to 0.74 nm, as converted to a binding thickness (the solid gray line at the top of Fig. 24). In contrast, the signal from the sample after isolation was measured to be only -0.03 nm (the solid black line at the bottom of Fig. 24). The same experiment was repeated for melanoma cancer samples obtained before and after isolation (see Fig. 25). The measured signals were 0.04 nm before isolation (the solid black line at the top of Fig. 25) and < 0.01 nm after isolation (the solid gray line at the bottom of Fig. 25). Comparing these results, the intensities of the signals from the CD9-containing exosomes in the normal and melanoma cancer standard samples before isolation showed large differences depending on the source of the samples, whereas the intensities of the signals after affinity isolation for CD63 were reduced almost to the level of background signals.

The same label-free sensing technique was applied to the analysis of the same samples using sensors immobilized with a Cavl-specific capture antibody. For a normal sample, the binding signal from the Cavl-containing exosomes only slightly increased from 0.04 nm before isolation to 0.06 nm after isolation (see Fig. 26). For a melanoma cancer sample, the binding signal from the Cavl-containing exosomes increased more than 10-fold from < 0.01 nm before isolation to 0.1 nm after isolation (Fig. 27). These results are thought to be due to the specific effect associated with affinity isolation for CD63.

In summary, the use of the inventive method based on immunoaffinity isolation led to a significant reduction in the concentration of CD9-containing exosomes regardless of the source of the exosome samples. In contrast, the inventive isolation method increased the concentration of Cavl-containing exosomes, particularly remarkably increased the concentration of Cavl-containing exosomes in the melanoma cancer sample. Such contrary effects of the inventive isolation method on the exosomal markers' concentration change provided a clear interpretation in terms of the underlying isolation mechanism.

The above results may actually vary depending on the progression stage of melanoma cancer disease when diagnosed. According to a published document, the concentration of Cav1+ exosomes, which was reported to increase during cancer development, increases significantly in the metastatic stage of cancer rather than in the early stage of cancer development. Therefore, the concentrations of Cav1+ exosomes in samples collected in the metastatic stage of cancer are easy to measure without exosome isolation and enrichment. However, the concentrations of Cav1+ exosomes in body fluids such as blood could be very low in the early stage of cancer development and are thus very difficult to directly measure even with sensitive analytical methods such as immunoassay. In conclusion, the isolation and enrichment of CD63+/Cav1+ exosome sub-subpopulations proposed in the present invention will allow for early and accurate cancer diagnosis through the analysis of biomarkers (for example, specific miRNAs such as miR222 for melanoma cancer) present in exosomes.

### III. Analysis of exosomes in clinical samples

### 1. Materials

Anti-CD63 antibody (Cat. No. 353014), anti-CD81 antibody (Cat. No. 349501), and anti-CD151 antibody (Cat. No. 350404) were purchased from BioLegend (San Diego, CA, USA) and anti-survivin antibody (Cat. No. NB500-201) was purchased from Novus Biologicals (Centennial, CO, USA). Monoclonal antibodies specific for CD9 (Cat. No. MAB1880) or Caveolin-1 (Cavl; Cat. No. MAB5736) were supplied by R&D Systems (Minneapolis, MN, USA). Samples (serum or plasma) from malignant melanoma and prostate cancer-positive patients were purchased from Discovery Life Sciences (Huntsville, AL, USA) and Promeddx (Norton, MA, USA). Other materials were the same as those used in the section "I. Affinity isolation of exosomes based on switch-like binding reaction".

### 2. Example 1: Preparation of CD63+ exosome subpopulation sample

### 2.1 Conjugation between SA and CBP

The procedure described in the section "I. Affinity isolation of exosomes based on switch-like binding reaction" was repeated.

### 2.2 Biotinylation of anti-CD63 antibody

The procedure described in the section "I. Affinity isolation of exosomes based on switch-like binding reaction" was repeated.

### 2.3 Pretreatment of clinical sample

A clinical sample (serum or plasma; 200 µl) was centrifuged at a low speed (1,200 g) at 4 °C for 20 min to remove the precipitate and centrifuged at a high speed (10,000 g) at the same temperature for 30 min to remove the precipitate. The supernatant was filtered through an HM syringe filter (Cat. No. SC13P020SL; 0.2 µm) to pretreat the clinical sample. The pretreated clinical sample was used as a bulk population sample.

### 2.4 Preparation of immunomagnetic beads

For magnetic enrichment, the amine groups on the CBP-specific antibody were chemically bound to and immobilized onto the activated tosyl moieties on the solid surfaces of the magnetic beads. After washing 3 times with Tris-HCl buffer, the remaining surfaces were blocked with casein-PBS. After magnetic separation of the beads (total 1 mg) from the solution, the CBP-SA conjugate (5 µg/ml; 1 ml) diluted with a BSA-Tris binding solution containing Ca²⁺ (10 mM) ("Ca²⁺ switch-on" condition) was added and incubated on a shaker for 1 h. After washing the beads with a BSA-free binding solution, the biotinylated anti-CD63 antibody (3 µg/ml; 1 ml) diluted with a BSA-Tris binding solution was incubated under the same conditions, followed by magnetic isolation.

### 2.5 Isolation of CD63 positive exosomes

CD63-positive (CD63+) exosomes were isolated and recovered using the immunomagnetic beads and the beads were regenerated. To isolate an exosome subpopulation of CD63+, which is a type of exosomal marker, the pretreated clinical sample (serum or plasma; 200 µl) was diluted 5-fold with a binding solution containing Ca²⁺ (10 mM) ("Ca²⁺ switch-on" condition) and added to the immunomagnetic beads. After incubation on a shaker for 1 h, magnetic isolation was performed. A Ca²⁺-containing binding solution was again transferred to wash off unbound components and then washed by magnetic separation. After that, a 20 mM Tris-HCl (pH 7.4) buffer solution containing only 500 mM NaCl ("Ca²⁺ switch-off' condition) was transferred and the CBP-antibody-exosome complex (containing the CD63+ exosome subpopulation) bound to the magnetic beads was recovered by magnetic separation. Thereafter, a 100 mM sodium acetate buffer (pH 4.5) containing 500 mM NaCl, 5% (v/v) Tween-20, and 0.02% (w/v) thimerosal was fed to the magnetic beads for regeneration, and the magnetic beads were thoroughly washed until no protein elution was observed.

### 3. Example 2: Profiling of exosome surface biomarkers

### 3.1 Enzyme-linked immunosorbent assay

To characterize the CD63+ exosome subpopulation separated by magnetic isolation, profiling was conducted on the biomarkers present on the exosome surfaces. To this end, antibodies specific to the biomarkers were immobilized onto wells of a microtiter plate and sandwich enzyme-linked immunosorbent assay (ELISA) was conducted on target biomarkers (CD9, CD81, Cavl, and survivin). Depending on the type of cancer disease, CD81, Cavl, and survivin, which are exosome biomarkers known to be involved in tumor progression, promotion or metastasis, and CD9, which is known as a tumor suppressor, were selected as the target biomarkers. First, antibodies specific to the biomarkers (5 µg/ml; 100 µl) were immobilized onto wells at 37 °C for 1 h and washed with deionized water. The remaining surfaces were treated with a 20 mM Tris-HCl (pH 7.4) buffer containing 0.5% casein under the same conditions as above. The CD63+ exosome subpopulation sample separated by magnetic isolation or the population sample before separation as a control was plated in the wells immobilized with the antibodies specific to the biomarkers, followed by incubation at 37 °C for 10 h, as described above. After washing, streptavidin-poly-HRP20 (66 ng/ml; 100 µl) was added, followed by incubation at room temperature for 1 h. After washing again, an HRP substrate solution (0.05 M acetate buffer solution containing 0.003% hydrogen peroxide and 100 µg/ml TMB, pH 5.1; 200 µl) was added, followed by incubation for 15 min. The reaction was stopped with the addition of a sulfuric acid solution (2 M; 50 µl). The color development in each well was quantified with an ELISA reader (Synergy H4, BioTek Inc.; Winooski, VT, USA) by the absorbance at 450 nm.

### 3.2 Analysis of correlations between biomarkers

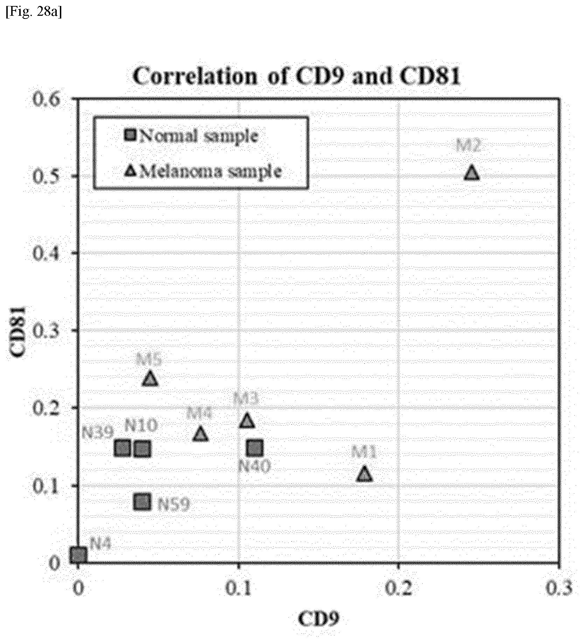

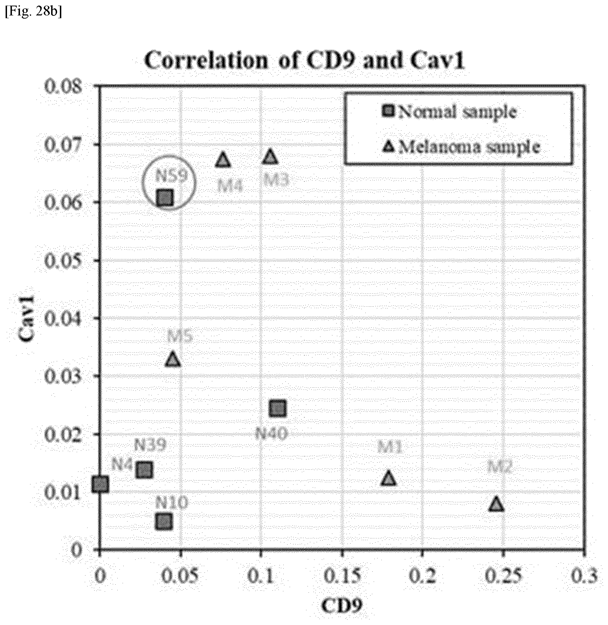

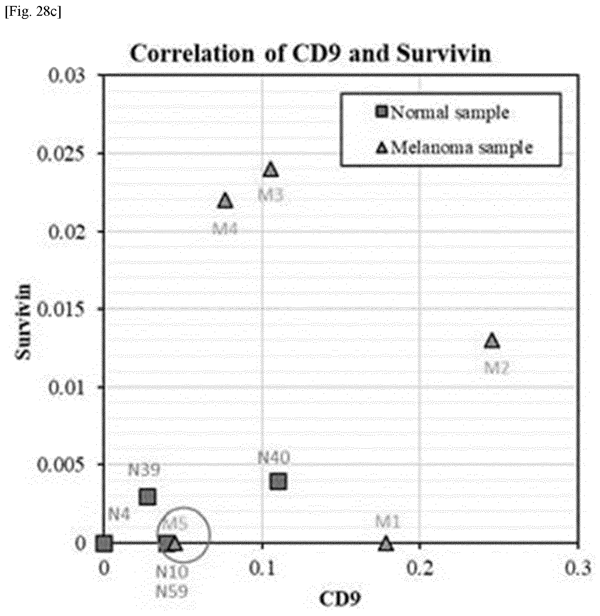

As described above, an immunoassay was conducted on CD81, Cavl, survivin, and CD9 using exosome bulk populations obtained by pretreatment of a malignant melanoma-positive clinical sample (serum or plasma) and a normal sample (serum). Here, since signals proportional to remaining immunocomplexes after reaction of each exosome bulk population sample with solid-phase capture antibodies specific to the biomarkers and separation were measured, a subpopulation containing the biomarkers was able to be quantitatively analyzed. Fig. 28 compares correlations between a marker for tumor progression, promotion or metastasis and a marker for tumor suppression after profiling of exosome surface markers in exosome bulk population samples from normal and malignant melanoma patient groups. In Fig. 28, the distributions of the samples were plotted two-dimensionally to obtain correlations between signals from CD81, Cavl, or survivin, as tumor progression, promotion or metastasis markers, and signals from CD9 as a tumor suppressor marker from quantitative signals obtained by ELISA. Referring to Fig. 28, when the exosome bulk populations were used as samples, data on the cancer-positive sample and data on the normal sample were intermixed in their distribution without a clear correlation therebetween. For reference, when the exosome bulk population samples were used, it was difficult to secure reliability for correlation analysis because the signal values from Cavl and survivin in most samples were very low.

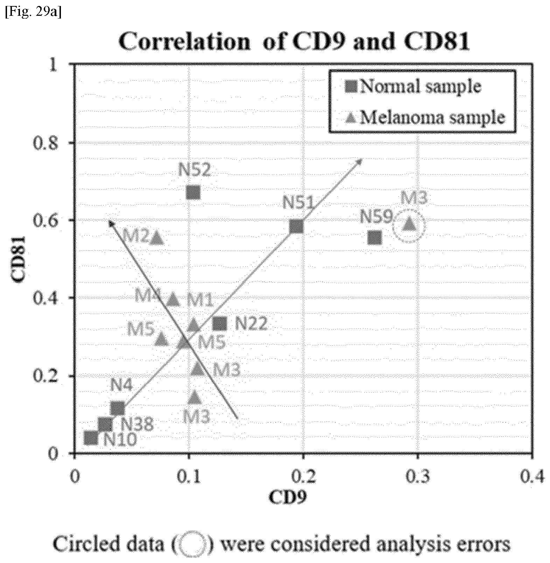

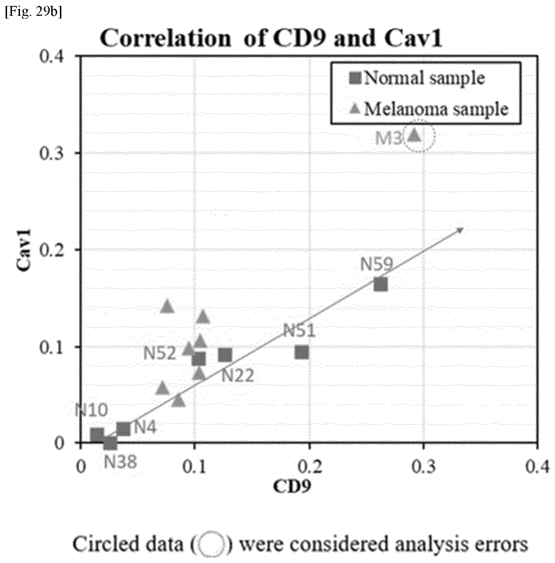

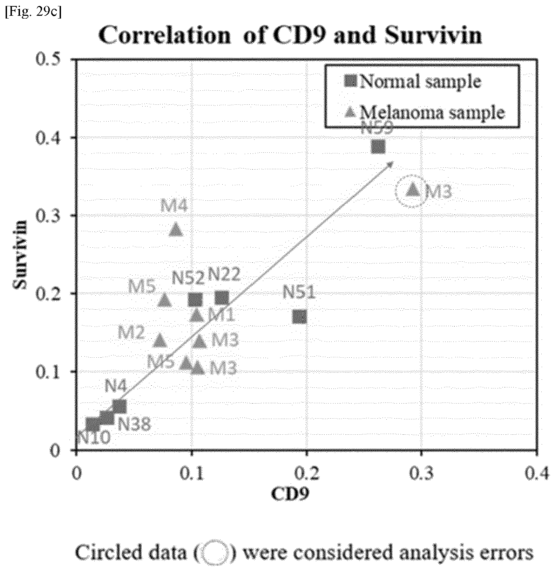

After the malignant melanoma positive and normal sample groups were pretreated, as described previously, CD63+ exosomes were separated and recovered using the reversible immunoisolation system based on "switch-like binding reaction" to prepare exosome subpopulation as analytical samples, and immunoassays were then performed on four types of biomarkers. A particular advantage of this immunoassay is that exosomes containing biomarkers present in the exosome subpopulations are isolated after reaction with specific antibodies immobilized on solid-state immobilization members and signals proportional thereto are generated from remaining immunocomplexes, enabling profiling of specific sub-subpopulations. Fig. 29 compares correlations between the markers for tumor progression, promotion and metastasis based on the marker for tumor suppression after profiling of the exosome surface markers in exosome sub-subpopulations using the exosome subpopulations separated from the exosome bulk populations from the normal and malignant melanoma patient groups as samples. In Fig. 29, the distributions of the samples were plotted two-dimensionally to obtain correlations between signals from CD81, Cavl, and survivin, as tumor progression, promotion or metastasis markers, and signals from CD9 as a tumor suppressor marker obtained by ELISA. In the first graph where signals from CD9, a tumor suppressor marker, were plotted versus signals from CD81, a tumor progression marker, the normal samples showed a linear proportional relationship between the two markers, indicating that the composition ratios of the two markers in the samples were kept constant. In contrast, the malignant melanoma-positive samples showed an inverse relationship between the two markers, indicating that the composition ratios of CD81 to CD9 in the cancer-positive samples were different.

Comparing the two results from the different sample groups, the heterogeneity of the composition ratio between the exosomal surface markers is predicted to increase when malignant melanoma occurs. Particularly, as cancer stage advanced from stage 1 to 4, the CD81 signal increased but the CD9 signal decreased, implying an increase in the CD81/CD9 signal ratio. Therefore, the increased heterogeneity of the composition ratio between the markers is predicted to be related to cancer stage.

Also in the second graph where signals from CD9 were plotted versus signals from Cavl and the third graph where signals from CD9 were plotted versus signals from survivin, the distributions of the cancer samples seem to deviate from the linear proportional relationship shown between the two markers in the normal samples. However, no clear increase in heterogeneity was observed, unlike that observed in the first graph.

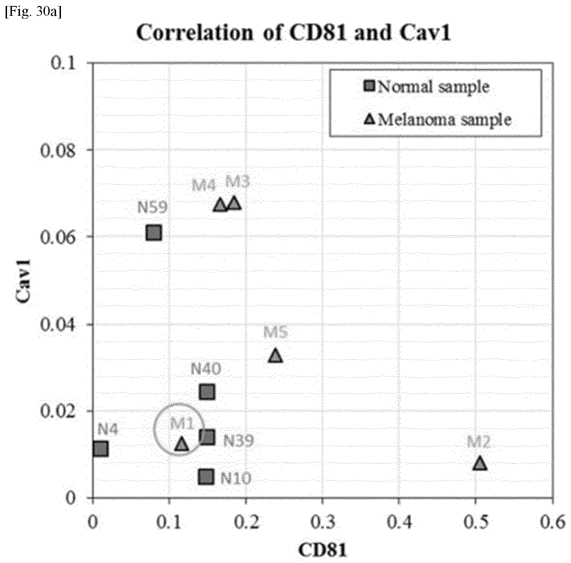

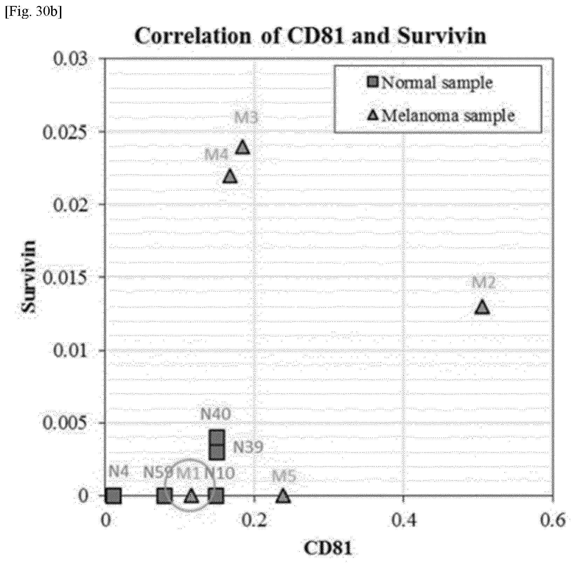

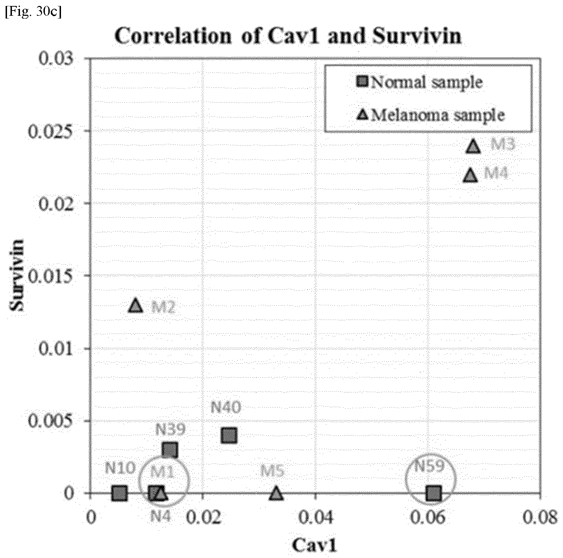

Fig. 30 compares correlations between markers for tumor progression, promotion and metastasis after profiling of exosome surface markers for exosome subpopulations in exosome bulk population samples from normal and malignant melanoma patient groups. As described above, an immunoassay was conducted on biomarkers using exosome bulk populations obtained by pretreatment of a malignant melanoma-positive clinical sample (serum or plasma) and a normal sample (serum). From the results, correlations between CD81, Cavl, and survivin as tumor progression, promotion or metastasis markers were analyzed. The analytical results showed that when the exosome bulk populations were used as samples, data on the cancer-positive sample and data on the normal sample were mixedly distributed without a clear correlation therebetween and it was difficult to secure reliability for correlation analysis because the signal values from Cavl and survivin in most samples were very low, as in Fig. 28.

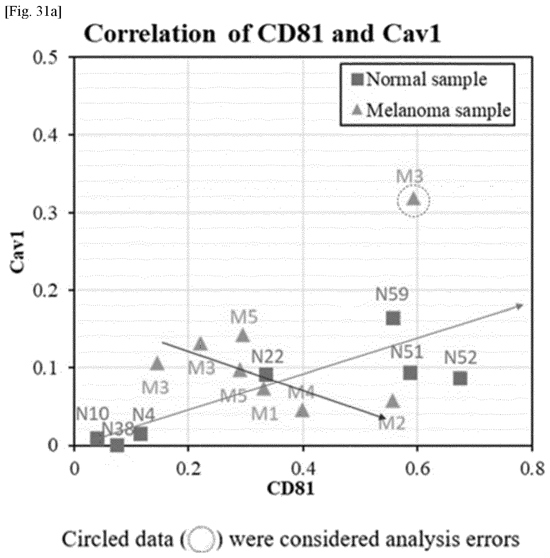

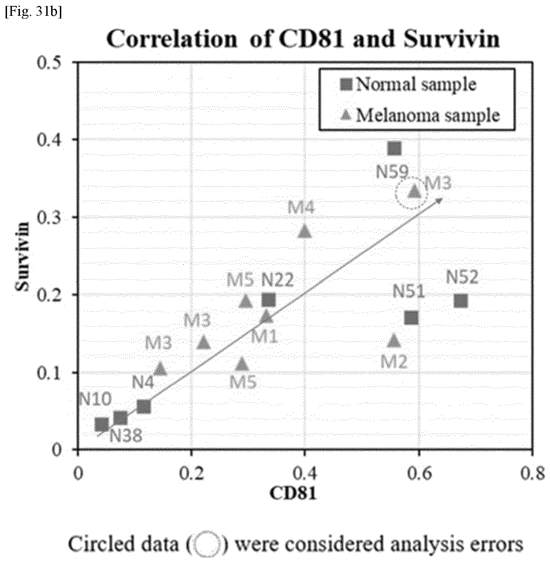

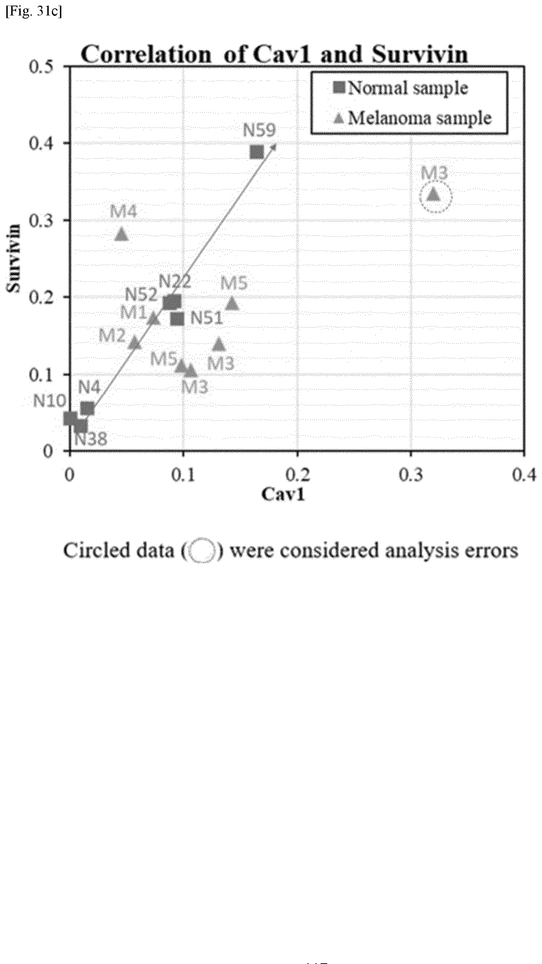

Fig. 31 compares correlations between markers for tumor progression, promotion and metastasis after profiling of exosome surface markers in exosome sub-subpopulations in exosome subpopulation samples separated from exosome bulk populations from normal and malignant melanoma patient groups. After the malignant melanoma positive and normal sample groups were pretreated, CD63+ exosome subpopulations were separated and recovered using the reversible immunoisolation system. After an immunoassay was conducted on biomarkers for the exosome sub-subpopulations in the same manner as above, correlations between three types of biomarkers for tumor progression, promotion and metastasis were analyzed. As can be seen from the immunoassay results, the normal samples showed a substantially linear proportional relationship between the two markers in the first graph where signals from CD81 were plotted versus signals from Cavl. In contrast, the malignant melanoma-positive samples showed an inverse relationship between the two markers, where the Cavl signals were higher than the CD81 signals in the early stage of cancer disease and reversed in the late stage.

This phenomenon was not observed in the second graph where signals from CD81 were plotted versus signals from survivin and the third graph where signals from Cavl were plotted versus signals from survivin. However, in the third graph, the distribution of the cancer samples seems to deviate from the linear proportional relationship between the two markers in the normal samples.

### 4. Example 3: Setting of parameters for early diagnosis of disease based on biomarker profiling results and analysis using the parameters

### 4.1 Setting of diagnostic parameters

Since the concentrations of exosomes in samples are different and vary depending on dilution factors, it is difficult to directly compare signal differences for biomarkers in samples calculated from immunoassay results. In an attempt to solve this problem, two or more types of normalized parameters for target diseases to be diagnosed were newly defined from the ratios between signals from one biomarker and signals from other biomarkers. Specifically, normalized parameters 1, 2, and 3 were set from the ratios of signals from CD81, Cavl, and survivin, markers for tumor progression, promotion or metastasis, to signals from CD9, a tumor suppressor marker. Here, parameters 1, 2, and 3 were set as 'signal from CD81 / signal from CD9', 'signal from Cavl / signal from CD9', and 'signal from survivin / signal from CD9', respectively.

### 4.2 Two-dimensional parameter analysis

After the results of profiling of exosome biomarkers in samples were converted to the parameters as defined above, an optimal pair of two parameters was selected for two-dimensional analysis. Based on the analytical results, the boundaries of the two parameter values were set such that normal and target disease samples could be distinguished from each other, and the abilities discriminating negative and positive samples for the target disease, that is, the positive accuracy (sensitivity) and negative accuracy (specificity), were determined. For reference, the parameter values of the boundary lines by which normal and target disease samples are distinguished from each other may vary depending on the analytical conditions.

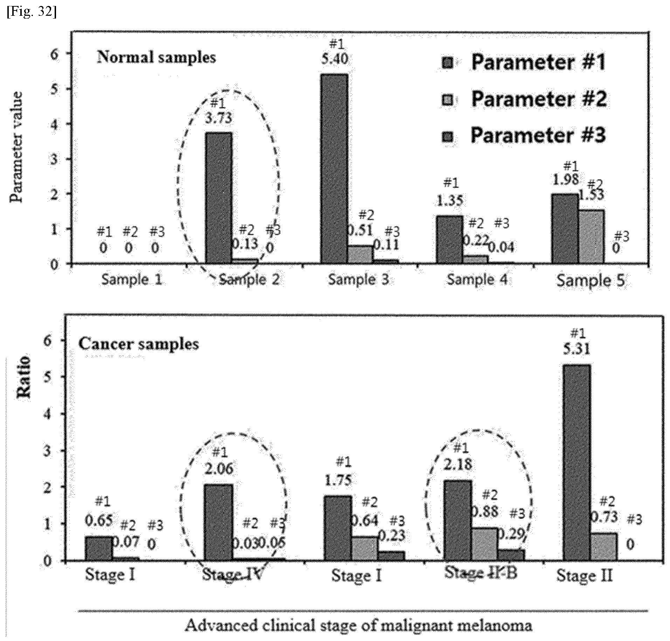

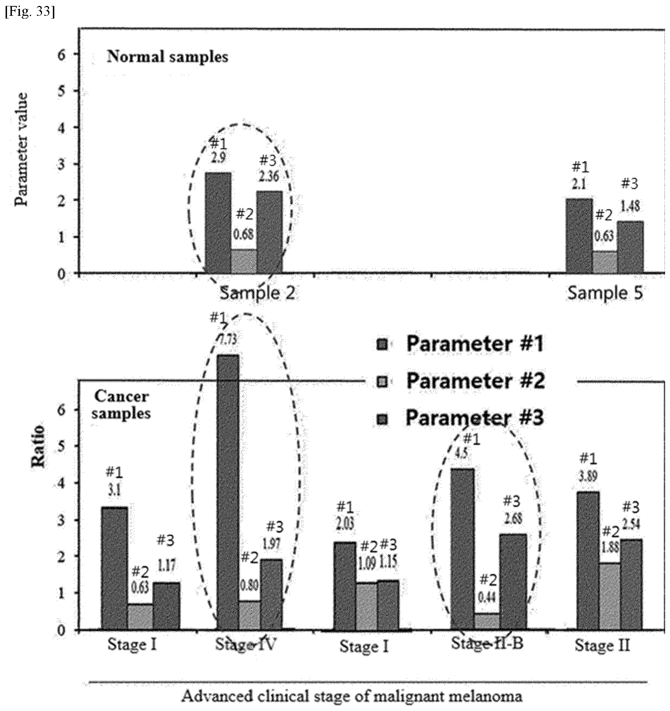

Fig. 32 shows the results of biomarker profiling for exosome subpopulations in exosome bulk population samples from normal and malignant melanoma patient groups when newly defined parameters were applied to the biomarker profiling. Fig. 33 shows the results of biomarker profiling for exosome subpopulation samples separated from exosome bulk populations from normal and malignant melanoma patient groups when newly defined parameters were applied to the biomarker profiling.

It was difficult to elucidate profiling differences between normal and cancer samples from the results of biomarker profiling for exosome subpopulations in exosome bulk population samples before immunoisolation (see Fig. 32) because the values of parameter 3 were too small regardless of the normal or cancer samples. This was because the results of immunoassay for survivin were measured too small in all population samples, as shown in Figs. 28 and 30.

Next, in the results of biomarker profiling for the exosome subpopulation samples obtained after immunoisolation and recovery (see Fig. 33), the values of the three parameters were high enough to compare the samples. Particularly, the values of all parameters increased noticeably, which enabled profiling of the selected exosome biomarkers when appropriate exosome subpopulations were separated, recovered, and used as analytical samples.

This effect is made possible by the synergistic action of reduced analysis observation windows and concentrated targets because isolated and recovered target exosomes were used as samples. As exosome species released from many different cells are mixed in body fluids, the use of exosome subpopulations containing target exosomes isolated and recovered from body fluids as samples can exclude irrelevant exosome species from disease-relevant exosome species. Thus, it will be effective in reducing observation windows in a desirable direction when the sizes of observation windows are compared. In this case, it is possible to minimize interference caused by non-specific reactions of irrelevant biomarkers during disease diagnosis. Furthermore, enrichment for separation into subpopulations leads to an increase in the concentration of target exosomes, achieving high recovery yield. Therefore, according to the present invention, the use of the exosome subpopulation as an immunoassay sample for diagnosis minimizes interference caused by non-specific exosomes and leads to an increase in the concentration of target exosomes, achieving a positive synergistic effect on the accuracy of profiling.

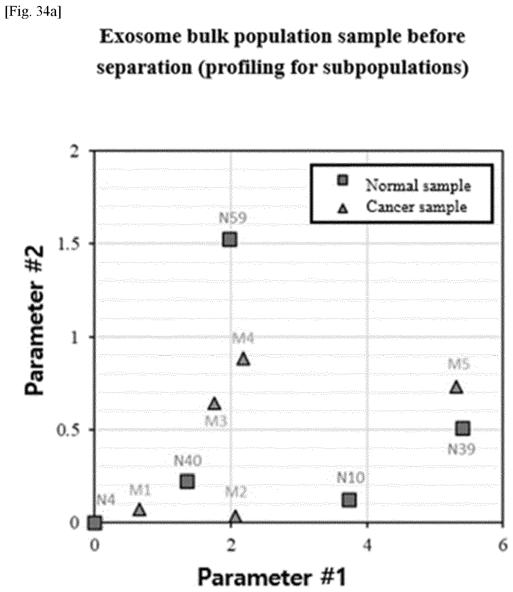

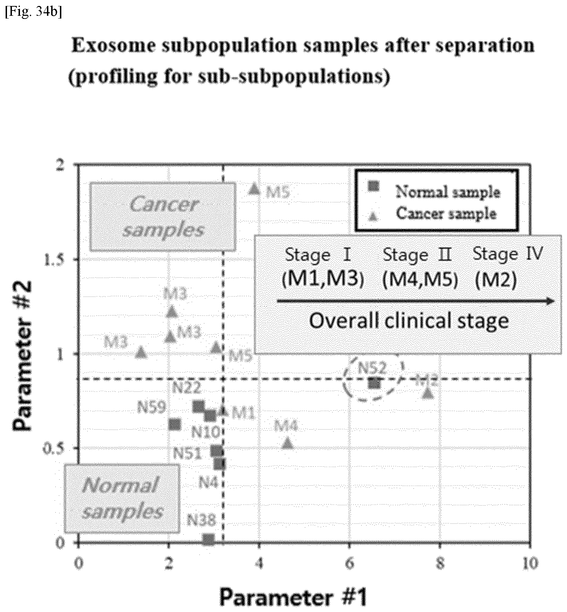

Fig. 34 shows the overall results of two-dimensional parameter analysis for the results of biomarker profiling for exosome subpopulations in exosome bulk population samples (A) from normal and malignant melanoma patient groups and for exosome sub-subpopulations in exosome subpopulation samples (B) separated from the exosome bulk populations when newly defined parameters were applied to the biomarker profiling.

To elucidate the effect of using exosome subpopulations recovered after immunoisolation as diagnostic samples, first, the results of profiling for the subpopulations containing the biomarkers in the exosome bulk population sample before separation (see Figs. 28 to 31) were converted into parameters defined in Figs. 32 and 33. First, two-dimensional analysis of parameters 1 and 2 was performed (see Fig. 34, A). As a result, it was found that the normal and cancer samples were mixedly distributed in the two-dimensional plot with no difference between the sample groups.

Next, the sub-subpopulations in the subpopulation samples obtained by recovery after immunoisolation were profiled and the results were converted into parameters in the same manner as above. Thereafter, two-dimensional analysis of parameters 1 and 2 was conducted (see Fig. 34, B). As a consequence, the normal samples were distributed in the limited area defined by the two parameter values (parameter 1 < ~3.2; parameter 2 < ~0.9) (see the boundary defined by the dashed lines in the graph) and the malignant melanoma samples were distributed on or outside the boundary. Only one (N52) of the normal samples was located outside the boundary, which is considered to be caused by an analysis error or an unknown sample at the time of analysis. Therefore, according to the present invention, normalization effects were observed by sequential immunoisolation, enabling discriminatory analysis according to the sample sources using disease-associated exosome biomarkers in sub-subpopulations. As a result, samples negative for cancer could be differentiated from samples positive for cancer. For reference, the parameter values of the boundary lines by which the cancer samples and the normal samples are distinguished from each other are exemplary and may vary depending on the analytical conditions.

It was also found that the stage of malignant melanoma disease could be predicted by two-dimensional analysis of parameters 1 and 2. Particularly, the analytical results of samples from stage I, II, and IV malignant melanoma patients showed that the value of parameter 1 increased proportionally with the stage of the disease. Notably, the parameter 1 values of the stage I and II melanoma cancer samples were distributed in the range of the values of the normal samples, but the parameter 2 values of the cancer samples were higher than the range of the values of the normal samples. These results are thought to be because the Cavl markers regulating the value of parameter 2 were involved in tumor suppression in the early stage of cancer development and were highly expressed. Therefore, it is believed that the two-dimensional analysis of parameters 1 and 2 allows to predict the stage of malignant melanoma and enables early diagnosis of early-stage cancer. In conclusion, the selection and analysis of multiple markers associated with a specific cancer disease in exosome subpopulations helps solve the problem that cancer is diagnosed in advanced or metastatic stage.

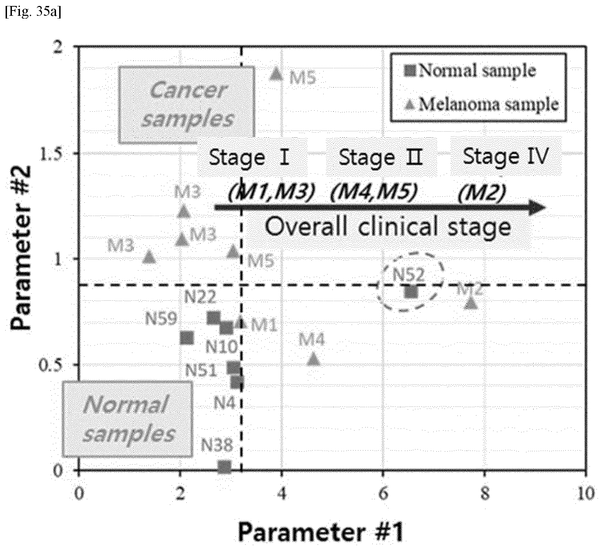

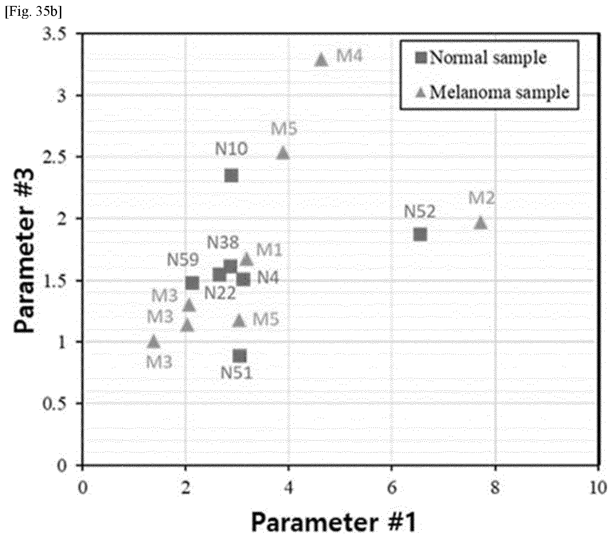

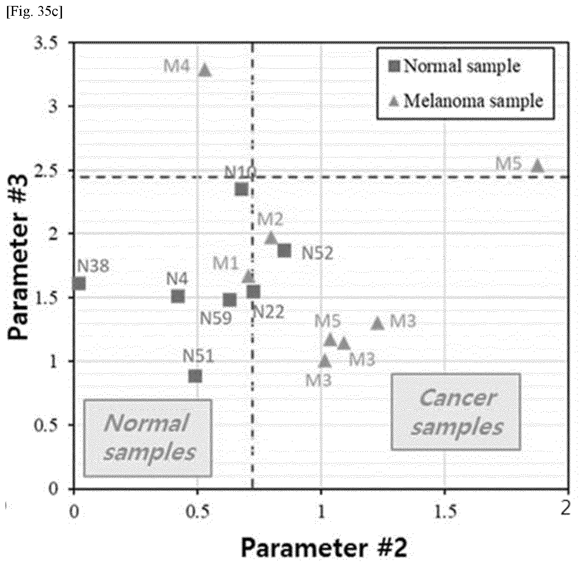

Fig. 35 shows the overall results of two-dimensional parameter analysis for the results of biomarker profiling for sub-subpopulations separated from exosome subpopulation samples from normal and malignant melanoma patient groups when newly defined parameters were applied to the biomarker profiling. In addition to the two-dimensional analysis of parameters 1 and 2 for the results of profiling for the exosome sub-subpopulations (see the left of Fig. 35), two-dimensional analyses of parameters 1 and 3 (see the middle of Fig. 35) and parameters 2 and 3 (see the right of Fig. 35) were conducted. The two-dimensional analysis of parameters 1 and 3 (see the middle of Fig. 35) revealed that the normal samples and the malignant melanoma samples were distributed in the graph without being distinguished from each other. Therefore, the analysis of parameters 1 and 3 is thought to fail to distinguish the normal samples from the malignant melanoma samples. In contrast, the analysis of parameters 2 and 3 (see the right of Fig. 35) was demonstrated to distinguish the normal samples from the malignant melanoma samples. The normal samples were distributed in the limited area defined by the two parameter values (parameter 2 < ~0.7; parameter 3 < -2.5) (see the boundary defined by the dashed lines in the graph) and the malignant melanoma samples were distributed on or outside the boundary. In conclusion, two-dimensional analysis of parameters 2 and 3 through profiling for exosome sub-subpopulations makes it possible to distinguish malignant melanoma samples from normal samples.

### 4.3 Parameter panel construction

To maximize the diagnostic sensitivity and specificity to the target disease, a panel composed of two or more parameters was used for early diagnosis of the disease. Specifically, two-dimensional analysis was conducted on pairs of parameters in the same manner as above. A pair of parameters was selected that provided the maximum ability to distinguish samples negative and positive for the target disease. The selected pair of parameters were used to construct a parameter panel. If necessary, one or more parameters were sequentially applied to the primarily constructed parameter panel to determine a final parameter panel that can distinguish negative and positive samples with maximum accuracy. A diagnostic algorithm using the final parameter panel was also determined.

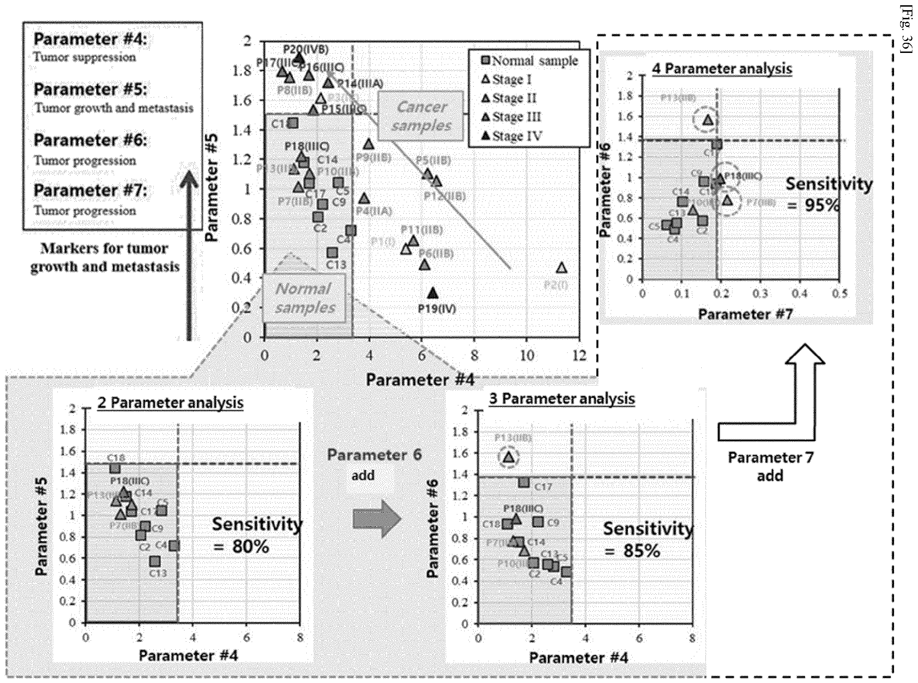

Fig. 36 shows the overall results of two-dimensional parameter analysis for the results of biomarker profiling for exosome sub-subpopulations in exosome subpopulation samples from normal and prostate cancer patient groups when newly defined parameters were applied to the biomarker profiling. For prostate cancer, CD151 was used as an additional exosome surface marker. CD63+ exosome subpopulation samples were profiled using a total of five biomarkers (CD9, CD81, Cav1, survivin, and CD151) in the same manner as described above. Similarly to above, normalized parameters 4, 5, 6, and 7 were newly defined by adopting ratios between signals from any two biomarkers where maximum clinical performance was achieved (total number of samples = 28; positive = 20, negative = 8). Here, parameters 4 to 7 were set as 'signal from CD9 / signal from Cav1', 'signal from CD151 / signal from CD9', 'signal from survivin / signal from CD9', and 'signal from Cav1 / signal from CD81', respectively.

First, two-dimensional analysis of parameters 4 and 5 was conducted on the results of profiling for exosome sub-subpopulations after immunoisolation and recovery of subpopulations of prostate cancer and normal samples. As a result of the two-dimensional analysis of parameters 4 and 5 (see the top of Fig. 36), most of the normal samples were found to be distributed in the limited area defined by the values of the two parameter values (parameter 4 < ~3.3; parameter 5 < ~1.5) under the test conditions (see the boundary defined by the dashed lines in the graph) and most of the prostate cancer samples were found to be distributed outside the boundary. When these two parameters were used, the positive accuracy *(i.e.* sensitivity) was 80% and the negative accuracy *(i.e.* specificity) was 100% under the analytical conditions.

Prostate cancer-positive samples (P7, P10, P13, and P18) located inside the above-mentioned boundary, *i.e.* false-negative samples, were precisely re-analyzed (see the bottom of Fig. 36). When two parameters *(i.e.* parameters 4 and 5) were used, the clinical sensitivity was limited to 80% (see the bottom left of Fig. 36). When two-dimensional analysis of parameter 4 with additional parameter 6 (a total of three parameters) was conducted, the negative samples remained in the negative area but one (P13) of the positive samples was shifted to the positive area (see the bottom middle of Fig. 36). As a result, the sensitivity was raised to 85%. When two-dimensional analysis of parameter 6 with additional parameter 7 (a total of four parameters) was conducted, the negative samples remained in the negative area but P7 and P18 of the positive samples were shifted to the positive area (see the bottom right of Fig. 36). As a result, the clinical sensitivity was increased to 95%. The final clinical performance for the prostate cancer samples was improved (sensitivity 95% and specificity 100%).

Furthermore, two-dimensional analysis of parameters 4 and 5 has been shown to predict the stage of prostate cancer. Particularly, as can be seen from the analytical results of patient samples according to the stage numbers (Roman numerals in the parentheses next to the sample numbers) of prostate cancer set by clinical judgment criteria (see the top of Fig. 36), 9 prostate cancer-positive samples were distributed in the lower right-hand corner defined by the boundary lines. Among them, 8 out of the 9 samples were positive in stage I and II and the remaining one was positive in stage IV. Another group of 7 prostate cancer-positive samples were distributed in the top lefthand corner defined by the boundary lines. Among them, 5 out of the 7 samples were positive in stage III and IV and the remaining two were positive in stage I and II. As the stage advanced, each sample decreased in inverse proportion to the value of parameter 4 and increased in proportion to the value of parameter 5 (see the arrow in Fig. 36). Notably, early-stage prostate cancer samples (stage I and II samples) were distributed in the bottom right-hand corner, that is, in the area where the value of parameter 4 was particularly high and the value of parameter 5 was low. Therefore, it is thought that sequential two-dimensional analysis of two or more normalized biomarkers not only helps predict the stage of prostate cancer but also enables early diagnosis of early-stage cancer.

### 5. Example 4: Profiling of exosome miRNA biomarkers

### 5.1 Cell line culture and exosome sample preparation

DU145, LNCaP, and PC3 cell lines, which are generally known prostate cancer-derived cell lines, were selected and cultured. Each of the cultures was harvested to prepare an exosome population sample, as in the clinical sample preparation described above. CD63+ subpopulation exosome samples were prepared from the exosome population based on "switch-like reversible binding reaction", as described above. CD63+/CD81+ sub-subpopulation samples were sequentially prepared from the CD63+ subpopulation exosomes by magnetic immunoisolation using magnetic beads immobilized with anti-CD81 antibody. HEK293, a cell line derived from kidney cells, was selected as a control and population, subpopulation, and sub-subpopulation exosome samples were prepared in the same manner as described above.

More specifically, human embryonic kidney-derived cell line HEK293 and prostate cancer-derived cell lines LNCaP, DU145, and PC3 were purchased from the Korea Cell Line Bank (Seoul, Korea). Each of the cell lines was cultured in an optimal medium supplemented with 10% fetal bovine serum (FBS) and antibiotics in a CO₂ incubator for 34-39 h. The medium was DMEM supplemented with HEPES for HEK293 or RPMI 1640 medium for the prostate cancer-derived cell lines. Thereafter, the medium was replaced with a medium supplemented with 5% exosome-free FBS and culture was performed under the same conditions. Cells were separated from the culture by centrifugation and the supernatant was collected. The supernatant was subjected to low-speed centrifugation to remove the precipitate and subsequent high-speed centrifugation to remove the precipitate, as in the pretreatment of the clinical sample described above. The resulting supernatant was filtered through an HM syringe filter (0.2 µm pore size) to prepare an exosome population sample.

CD63+ subpopulation exosome samples were prepared from the exosome population by immunomagnetic isolation in the same manner as in Example 1. Anti-CD81 antibody was chemically immobilized onto magnetic beads following the protocol provided by the manufacturer of the magnetic beads. CD63+/CD81+ sub-subpopulation samples were prepared from the CD63+ exosome subpopulation by immunomagnetic isolation using the magnetic beads chemically immobilized with anti-CD81 antibody.

### 5.2 Exosome miRNA extraction, cDNA synthesis and real-time PCR

Four types of prostate cancer-related miRNAs, *i.e.* miR-17, miR-21, miR-221, and miR-375, were selected as target nucleic acid biomarkers. After extraction of the miRNA markers from each exosome sample, cDNA was synthesized by reverse transcription. The products were amplified by polymerase chain reaction. For each sample, the cycle threshold (Ct) value was sensitively measured as a signal. The overall procedure was performed using commercially available kits. For normalization, the ratios of signals from two miRNA markers, that is, the ratios of 'signal from miR-21 / signal from miR-17', 'signal from miR-221 / signal from miR-17', and 'signal from miR-375 / signal from miR-17', were calculated.

Specifically, three types of exosome samples *(i.e.,* population, subpopulation, and sub-subpopulation samples) were prepared for each cell line, and miRNAs were extracted from the samples using miRNeasy Micro Kit (QIAGEN, 217084). Each step was carried out according to the manufacturer's instructions. The extracted miRNAs were quantified using a spectrophotometer (ND-2000; NanoDrop Technologies, Wilmington, DE, USA). Next, reverse transcription of the miRNAs (2 ng) extracted from the exosome samples was performed on a 15 µl reaction volume scale using a TaqMan Advanced miRNA cDNA Synthesis kit (Applied Biosystems, Grand Island, NY, USA) according to the manufacturer's instructions. Thereafter, miRNA amplification was performed and real-time PCR was performed quantitatively. Quantitative PCR of target miRNAs (miR-17, miR-21, miR-221, and miR-375) was performed using TaqMan microRNA Assay kits (Applied Biosystems). Quantification was performed using CFX 384 (Bio-Rad Laboratories, CA, USA). The miRNAs in each sample were measured repeatedly. Each Ct value was normalized to miR-17 and scaled with respect to the signal ratio in the exosome population of the HEK293 cell line as a control, based on which the fold change was calculated.

### 5.3 Analysis of exosomal miRNA biomarkers in samples

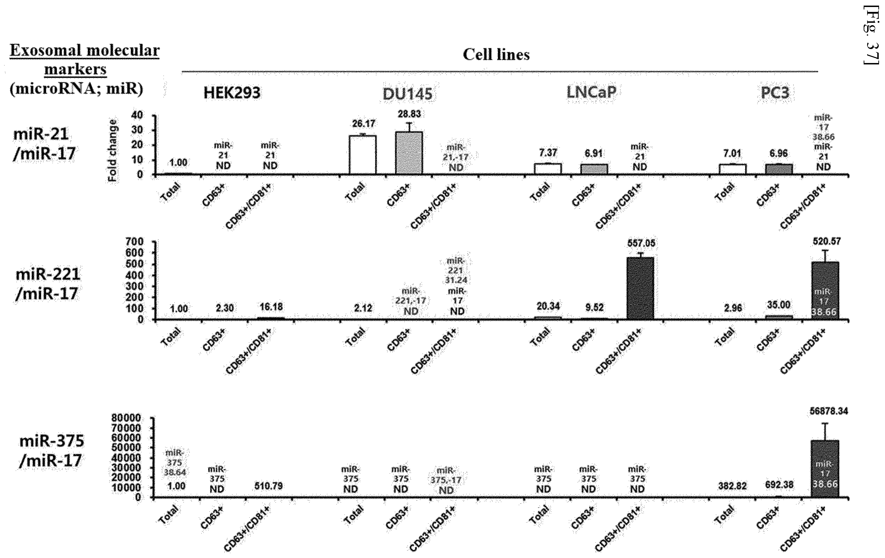

For the exosome population, subpopulation, and sub-subpopulation samples prepared from each cell line, changes in the signal ratio between the three types of miRNAs defined above were monitored. Fig. 37 shows the analytical results for prostate cancer miRNA biomarkers present in the exosome population, CD63+ subpopulation, and CD63+/CD81+ sub-subpopulation samples from the cultures of the prostate cancer-derived cell lines. For easy comparison, all signal ratios were scaled with respect to each signal ratio in the exosome population of the HEK293 cell line as a control, based on which the fold change was calculated and plotted.

First, the exosome population and subpopulation samples from all three prostate cancer cell lines showed significantly increased miR-21/miR-17 signal ratios based on those samples from the control cell line HEK293, *i.e.* fold change (see the top of Fig. 37). However, the miR-21/miR-17 signal ratios were not increased or detected in the sub-subpopulation samples. Particularly, the fold increases were pronounced in the samples from the DU145 cell line, indicating a specific association with the miR-21 biomarker. As another specific feature, miR-21 was not measured in the CD63+/CD81+ sub-subpopulation exosomes from all selected cell lines. Therefore, it is predicted that there is an inverse correlation between the exosome sub-subpopulation surface markers used for immunoisolation and the miRNAs present in exosomes.

Next, the miR-221/miR-17 signal ratios in the samples from the DU145 cell line were similar to those in the population of the control cell line regardless of the exosome populations (see the middle of Fig. 37). The signal ratios in population and subpopulation of the LNCaP and PC3 cell lines showed no substantial fold increases. However, the fold increases were pronounced in the sub-subpopulation exosomes from the LNCaP and PC3 cell lines. These results were contrary to the results for miR-21/miR-17 signal ratios, demonstrating a proportional association between the exosome sub-subpopulation surface markers and the miRNAs present in the exosomes.

Finally, for the miR-375/miR-17 signal ratios (see the bottom of Fig. 37), no fold increases were observed in all exosome samples from DU145 and LNCaP, like in the samples from the control. In contrast, fold increases were observed in the samples from the PC3 cell line. Particularly, a significant fold increase was observed in the sub-subpopulation sample. Since the PC3 cell line was derived from prostate cancer with a relatively high degree of malignancy, it is predicted that miR-375 present in the CD63+/CD81+ sub-subpopulation exosomes may be used as a marker associated with cancer progression. Therefore, the use of an exosome sub-subpopulation as an analytical sample for a specific miRNA is expected to help select a specific biomarker depending on the type of cells.

To conclude, according to the present invention, when proteins and miRNAs present in exosome sub-subpopulation associated with cancer diseases such as malignant melanoma and prostate cancer are selected as biomarkers and converted into normalized parameters for analysis, samples negative and positive for the cancer diseases can be distinguished and the cancer diseases can be diagnosed at the early stage. According to the present invention, effective parameters for diagnosis or combinations thereof are predicted to vary depending on the type of cancer disease. For reference, since the parameter values to distinguish samples negative and positive for cancer disease in Figs. 35 and 26 may vary depending on analytical conditions, they do not have absolute meanings. The distributions of the cancer samples relative to the normal samples in the graphs, which are obtained through two-dimensional (in some cases, one-dimensional) analysis of the parameters, are meaningful.

Although the present invention has been described herein with reference to the specific embodiments, these embodiments do not serve to limit the invention and are set forth for illustrative purposes. It will be apparent to those skilled in the art that modifications and improvements can be made without departing from the spirit and scope of the invention.

Such simple modifications and improvements of the present invention belong to the scope of the present invention, and the specific scope of the present invention will be clearly defined by the appended claims.

### Industrial applicability

According to the present invention, a subpopulation, a sub-subpopulation or a subset thereof containing exosomes associated with a specific disease in a body fluid can be separated and recovered intact in high yield to prepare a liquid biopsy sample and the liquid biopsy sample can be analyzed. Therefore, the present invention can find application in various industrial fields, including *in vitro* diagnosis, therapeutic development, and beauty industry.

## Claims

1. A device for preparing an exosome liquid biopsy sample comprising solid-state immobilization members, first capture materials specifically binding with first separation markers of first target exosomes associated with a predetermined disease in a bulk population sample containing exosomes released from cells to capture the first target exosomes, and first reversible linkers through which the first capture materials detachably bind to the immobilization members.

2. The device according to claim 1, further comprising second capture materials binding to the immobilization members to which the first capture materials are not bound and specifically binding with second separation markers of second target exosomes in an exosome subpopulation, which is a population of the first target exosomes, to capture the second target exosomes.

3. The device according to claim 2, further comprising second reversible linkers through which the second capture materials detachably bind to the immobilization members.

4. The device according to claim 3, wherein each of the first reversible linkers and/or the second reversible linkers comprises a ligand, a binder detachably binding with the ligand and whose conformation is changed when bound with the ligand, and a recognition material specifically binding to the binder whose conformation has been changed and separated from the binder when the ligand is detached from the binder and wherein one of the binder and the recognition material is immobilized on the surface of the immobilization member and the other is conjugated with the capture material to form a conjugate.

5. The device according to claim 4, wherein the ligand is selected from the group consisting of sugar molecules, ions, substrates, antigens, peptides, vitamins, growth factors, hormones, and combinations thereof.

6. The device according to claim 4, wherein the binder is selected from the group consisting of sugar-binding proteins, ion-binding proteins, enzymes, antibodies, avidins, aptamers, cell receptors, nanostructures, and combinations thereof.

7. The device according to claim 4, wherein the recognition material is selected from the group consisting of antibodies, protein receptors, cell receptors, aptamers, enzymes, nanoparticles, nanostructures, heavy metal chelators, and combinations thereof.

8. The device according to claim 3, wherein each of the first reversible linkers and/or the second reversible linkers comprises: a ligand conjugated with one of the capture material and the immobilization member to form a conjugate; and a binder immobilized onto the other of the capture material and the immobilization member, binding with the ligand, and separated from the ligand by competing with a competitive ligand.

9. The device according to claim 8, wherein the binder is selected from the group consisting of divalent metal ions, avidins, sugar-binding proteins, ion-binding proteins, enzymes, antibodies, aptamers, protein receptors, cell receptors, nanostructures, and combinations thereof; the ligand is selected from the group consisting of polyhistidine-tags (His-tags), vitamins, sugar molecules, ions, substrates, antigens, amino acids, peptides, nucleic acids, growth factors, hormones, and combinations thereof; and the competitive ligand is selected from the group consisting of imidazoles, vitamins, sugar molecules, ions, substrates, antigens, amino acids, peptides, nucleic acids, growth factors, hormones, and combinations thereof.

10. The device according to claim 1, wherein the immobilization members are selected from the group consisting of hydrogels, magnetic beads, latex beads, glass beads, nanometal structures, porous membranes, non-porous membranes, and combinations thereof.

11. The device according to claim 1, further comprising a reactor accommodating the immobilization members therein.

12. The device according to claim 1, wherein the immobilization members are concentrated using magnetic force, gravitational force, and/or centrifugal force.

13. A method for preparing an exosome liquid biopsy sample comprising (a) allowing first capture materials specifically binding with first separation markers of first target exosomes associated with a predetermined disease to bind to solid-state immobilization members through first reversible linkers, (b) allowing a bulk population sample containing exosomes released from cells to react with the first capture materials bound to the immobilization members such that the first capture materials capture the first target exosomes to separate a subpopulation containing the first target exosomes, and (c) dissociating the first reversible linkers such that the captured first target exosomes are separated from the immobilization members to recover the exosome subpopulation.

14. The method according to claim 13, further comprising (d) allowing second capture materials specifically binding with second separation markers of second target exosomes in the exosome subpopulation to bind to the immobilization members and (e) allowing an exosome subpopulation sample containing the recovered exosome subpopulation to react with the second capture materials bound to the immobilization members such that the second capture materials capture the second target exosomes to separate a sub-subpopulation of the second target exosomes.

15. The method according to claim 14, wherein the second capture materials bind to the immobilization members through second reversible linkers in step (d) and the method further comprises (f) dissociating the second reversible linkers such that the captured second target exosomes are separated from the immobilization members to recover the exosome sub-subpopulation.

16. The method according to claim 14, further comprising concentrating the immobilization members by which the first target exosomes are captured using magnetic force, gravitational force, and/or centrifugal force between steps (b) and (c).

17. The method according to claim 14, further comprising (g) concentrating the immobilization members by which the second target exosomes are captured using magnetic force, gravitational force, and/or centrifugal force.

18. A method for analyzing exosome liquid biopsy samples comprising (a) preparing exosome liquid biopsy samples containing target exosomes associated with a predetermined disease from exosome bulk population samples containing exosomes released from cells in normal and patient groups, (b) measuring quantitative signals from a plurality of biomarkers of the target exosomes present in the exosome liquid biopsy samples, and (c) analyzing the exosome liquid biopsy samples from the normal and patient groups according to correlations between the plurality of biomarkers based on the measured quantitative signal values.

19. The method according to claim 18, wherein, in step (c), each of the exosome liquid biopsy samples is analyzed according to a correlation between the quantitative signal from one biomarker and the quantitative signal from another biomarker.

20. The method according to claim 18, wherein step (c) comprises setting a plurality of parameters as ratios between the quantitative signal from one of the plurality of biomarkers as a reference signal and the quantitative signals from the other biomarkers and analyzing the exosome liquid biopsy samples according to correlation between two different parameters.

21. The method according to claim 20, wherein the setting of the parameters comprises: coordinating the exosome liquid biopsy samples in a plurality of XY coordinate systems, whose X-axis shows the quantitative signal from one of the plurality of biomarkers and Y-axis shows the quantitative signal from another biomarker, according to the signal values; calculating the slopes of the exosome liquid biopsy samples from the normal and patient groups; and setting the parameters using the biomarkers constituting the XY coordinate system in which the difference between the slope of the exosome liquid biopsy sample from the normal group and the slope of the exosome liquid biopsy sample from the patient group is relatively large.

22. The method according to claim 20, wherein the analysis of the exosome liquid biopsy samples comprises: in a first XY coordinate system whose X-axis shows one of the plurality of parameters as a first parameter and Y-axis shows another parameter as a second parameter, substituting the signal values for the first and second parameters to calculate the parameter values and coordinating the exosome liquid biopsy samples according to the calculated parameter values; in the first XY coordinate system, setting negative area in which the exosome liquid biopsy sample from the normal group is distributed and positive area; and further analyzing the coordinated liquid biopsy sample in the negative or positive area according to a correlation between the two parameters other than at least one of the first and second parameters.

23. The method according to claim 18, wherein the biomarkers comprise predetermined disease-associated biomarkers arranged on the surface of the exosomes and/or predetermined disease-associated biomarkers eluted from the exosomes.
